(19) Europäisches Patentamt
European Patent Office
Office européen des brevets

(11) **EP 3 033 649 B1**

(12) **EUROPÄISCHE PATENTSCHRIFT**

(45) Veröffentlichungstag und Bekanntmachung des Hinweises auf die Patenterteilung:
**03.03.2021   Patentblatt 2021/09**

(21) Anmeldenummer: **14747378.9**

(22) Anmeldetag: **04.08.2014**

(51) Int Cl.:
**G02B 27/00** (2006.01)    **A61F 2/16** (2006.01)
**G02C 7/04** (2006.01)

(86) Internationale Anmeldenummer:
**PCT/EP2014/066704**

(87) Internationale Veröffentlichungsnummer:
**WO 2015/022218 (19.02.2015 Gazette 2015/07)**

(54) **MULTIFOKALE AUGENLINSE MIT ZUMINDEST TEILWEISE UM EINE OPTISCHE HAUPTACHSE UMLAUFENDEN OPTISCHEN ZONEN**

MULTIFOCAL EYE LENS WITH OPTICAL ZONES WHICH AT LEAST PARTLY ENCIRCLE A MAIN OPTICAL AXIS

CRISTALLINS OCULAIRES MULTIFOCAUX PRÉSENTANT DES ZONES OPTIQUES ENTOURANT AU MOINS EN PARTIE UN AXE PRINCIPAL OPTIQUE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **13.08.2013   DE 102013216015**

(43) Veröffentlichungstag der Anmeldung:
**22.06.2016   Patentblatt 2016/25**

(73) Patentinhaber: **Carl Zeiss Meditec AG**
**07745 Jena (DE)**

(72) Erfinder: **GERLACH, Mario**
**16548 Glienicke-Nordbahn (DE)**

(74) Vertreter: **Hofstetter, Schurack & Partner**
**Patent- und Rechtsanwaltskanzlei**
**PartG mbB**
**Balanstrasse 57**
**81541 München (DE)**

(56) Entgegenhaltungen:
**WO-A1-2011/102719      DE-A1-102010 018 436**
**DE-A1-102011 101 899      DE-A1-102011 114 752**

**Beschreibung**

Technisches Gebiet

Die Erfindung betrifft multifokale Augenlinsen mit einem optischen Teil, der eine in Richtung einer optischen Hauptachse der Augenlinse betrachtet erste optische Seite und eine gegenüberliegende zweite optische Seite aufweist. Der optische Teil der multifokalen Augenlinse umfasst eine Mehrzahl von um die optische Hauptachse zumindest teilweise umlaufende, auf zumindest einer Seite ausgebildete, ringförmige optische Zonen, wobei Zonen jeweils zumindest eine Hauptunterzone aufweisen. Zwischen zwei in radialer Richtung benachbart und aneinander angrenzend ausgebildeten Zonen ist ein Phasenhub ausgebildet.

Stand der Technik

Multifokallinsen sind aus der DE 600 16 219 T2 bekannt. Bei den dortigen Multifokallinsen ist vorgesehen, dass um die optische Hauptachse vollständig umlaufende ringförmige Zonen ausgebildet sind. Jede Zone weist eine Hauptunterzone und eine Phasenunterzone auf. Zwischen zwei in radialer Richtung benachbarten Zonen ist ein Phasenhub ausgebildet. Die Zonen weisen gleiche Flächen auf und zwischen benachbarten Zonen ist keine optische Stufe ausgebildet, was bedeutet, dass die Oberflächengestaltung stetig ist. Insbesondere bedeutet dies auch, dass eine Wellenfront hinter der Linse stetig ist, das heißt, dass keine optischen Weglängendifferenzen beziehungsweise optischen Stufen zwischen Teilbereichen der Wellenfront hinter der Linse auftreten.

Durch die Phasenunterzonen kann Interferenzproblemen, wie sie in dem Stand der Technik auch erläutert sind, entgegengewirkt werden. Phasenverschiebungen zwischen Partialwellen aus verschiedenen Bereichen oder Zonen der Linse, bei denen die Phasenverschiebungen Voraussetzungen für eine Multifokalität der Linse sind, werden hier, nicht wie bei diffraktiven Linsen üblich, durch optische Stufen verursacht, sondern durch diese Phasenunterzonen mit spezifischer Brechkraft. Eine derartige Ausgestaltung und ein entsprechender Aufbau einer Linse mit dieser Unterteilung von ringförmigen Zonen in eine Hauptunterzone und eine Phasenunterzone stellen daher einen grundsätzlich unterschiedlichen Ansatz dar.

Aus der DE 10 2011 114 752 A1 und der DE 10 2011 101 899 A1 sind Intraokularlinsen bekannt, bei denen auf herkömmlichen diffraktiven Strukturen, die Zonen mit diskreten Stufen zwischen den Zonen aufweisen, weitere optisch abbildende Oberflächenstrukturen in Form von Helixwindungen überlagert sind.

Darüber hinaus ist aus der DE 10 2010 018 436 A1 eine weitere Ausgestaltung einer multifokalen Linse bekannt, bei der ebenfalls das Prinzip der ringförmigen Zonen, von denen Zonen eine Hauptunterzone und eine Phasenunterzone aufweisen, gezeigt ist.

Wie in dem Stand der Technik angegeben, gilt für eine gemittelte Brechkraft der Linse nachfolgender Zusammenhang:

$$F_{AV} = ( 1 - p)\, F_G + p\, F_S \qquad (1)$$

wobei dabei gilt, dass $F_{AV}$ eben die gemittelte Brechkraft ist, $F_G$ eine Brechkraft einer Hauptunterzone einer betrachteten Zone, $F_S$ die Brechkraft in der Phasenunterzone dieser Zone und p der Flächenanteil der Hauptunterzone an der gesamten Zone ist.

Des Weiteren ergibt sich die Differenz $\Delta F$ zwischen der größeren Brechkraft F2 (Nahbrechkraft) und der kleineren Brechkraft $F_1$ (Fernbrechkraft) auch aus der Additionsbrechkraft der zumindest bifokalen Linse, die aus ringförmigen Zonen mit jeweils zumindest einer Hauptunterzone und zumindest einer Phasenzone ausgebildet ist zu

$$\Delta F = \frac{\lambda N}{B^2} \qquad (2)$$

In dieser Formel 2 ist $\lambda$ die Design-Wellenlänge, die beispielsweise zwischen 540 und 560 nm, beispielsweise 546 nm betragen kann. N ist die Anzahl der ringförmigen Zonen, und B ist der Durchmesser der Linse, auf der sich die ringförmigen Zonen befinden und somit insbesondere der Durchmesser des optischen Teils der Linse.

Diese bekannten multifokalen Linsen weisen sehr gute Abbildungseigenschaften auf.

Aus der DE 10 2005 028 933 A1 ist eine astigmatische Intraokularlinse bekannt, die somit eine torisch brechende Linsenoberfläche aufweist.

Das menschliche Auge kann aufgrund seines relativ komplexen Aufbaus und somit auch insbesondere der zur optischen Abbildung vorgesehenen Teile und/oder anderweitiger Einflussfaktoren mit unterschiedlichsten Sehfehlern behaftet sein. Diese können individuell in der Stärke unterschiedlich ausgeprägt sein, andererseits auch eine Mehrzahl unterschiedliche Sehfehler an einem Auge vorliegen.

Um diese Sehfehlerkomplexität zumindest wesentlich verbessern zu können, ist es stetiges Bestreben, Augenlinsen dahingehend weiterzuentwickeln. Gerade bei Intraokularlinsen sind in dem Zusammenhang auch bezüglich der Handhabung bei einem operativen Eingriff die Kleinschnitttauglichkeit und somit das Einführen einer entsprechend gefalteten Intraokularlinse durch einen möglichst kleinen Schnitt im Auge, als auch die Kompaktheit einer derartigen Linse ebenso wesentliche Aspekte, die zu berücksichtigen sind.

Unter Berücksichtigung all dieser Anforderungen müssen somit neben einer Vielzahl und teils schwierig in Griff zu bekommenden optischen Anforderungen auch entsprechende Handhabbarkeitsanforderungen erfüllt werden.

Darstellung der Erfindung

Es ist Aufgabe der vorliegenden Erfindung, eine multifokale Intraokularlinse zu schaffen, die in ihren optischen Abbildungseigenschaften weiter verbessert ist.

Diese Aufgabe wird durch Multifokallinsen gemäß den unabhängigen Ansprüchen gelöst.

Gemäß einem ersten Aspekt der Erfindung umfasst eine multifokale Intraokularlinse einen optischen Teil, der für die optische Abbildungseigenschaft der Intraokularlinse charakteristisch und verantwortlich ist. Der optische Teil weist eine in Richtung einer optischen Hauptachse der Intraokularlinse betrachtet erste optische Seite und eine gegenüberliegende zweite optische Seite auf. Die erste Seite kann die Vorderseite oder die Rückseite des optischen Teils sein. Entsprechend kann dann die zweite Seite die ergänzende Rückseite oder Vorderseite sein. Die optische Hauptachse steht senkrecht auf einer axial zwischen der ersten und der zweiten Seite, insbesondere der Vorderseite und der Rückseite, liegenden Ebene der Intraokularlinse, insbesondere des optischen Teils, so dass die Seiten gegenüberliegend zu dieser Ebene entlang der Hauptachse betrachtet angeordnet sind.

Die Intraokularlinse umfasst darüber hinaus eine Mehrzahl von um die optische Hauptachse zumindest teilweise umlaufende, auf zumindest einer Seite ausgebildete, ringförmige optische Zonen auf, wobei Zonen jeweils zumindest eine Hauptunterzone aufweisen. Ein wesentlicher Gedanke der Erfindung ist darin zu sehen, dass die Intraokularlinse gemäß dem ersten Aspekt zusätzlich zu den ringförmigen Zonen eine Helixwindung als optische Oberflächenstruktur aufweist, so dass auch diese Helixwindung zur optischen Abbildungseigenschaft der Augenlinse beiträgt. Durch die Helixwindung ist eine Brechkraft der Intraokularlinse in Umlaufrichtung um die Achse wertmäßig variierend, wobei die Helixwindung auf zumindest einer Seite des optischen Teils ausgebildet ist. Durch eine derartige Ausgestaltung einer multifokalen Intraokularlinse ist die Schärfentiefe verbessert, so dass neben einem scharfen Sehen in zumindest zwei Foki und somit zwei spezifischen Brechkräften der Intraokularlinse auch die Schärfentiefe wesentlich verbessert ist. Dadurch tritt auch eine entsprechende Verbesserung gegenüber den bekannten Linsen aus dem Stand der Technik diesbezüglich auf.

Bei dem ersten Aspekt der Erfindung sind somit multifokale Augenlinsen umfasst, bei denen die ringförmigen optischen Zonen teilweise umlaufend ausgebildet sind und somit keine vollständig geschlossenen Ringe darstellen. Sie können jedoch auch vollständig geschlossene Ringzonen sein.

Insbesondere ist durch die Helixwindung ein Brechkraftanteil zur Gesamtbrechkraft der Linse beitragend, der sich in Umlaufrichtung um die optische Achse und somit azimutal wertmäßig kontinuierlich ansteigend verändert und somit insbesondere von einem Windungsanfang zu einem Windungsende der Helixwindung wertmäßig erhöht, insbesondere kontinuierlich erhöht. Insbesondere ist diese Brechkraftvariation, wie sie durch die Helixwindung beigetragen wird, bei einem vollständigen Umlauf um die optische Achse ohne Zwischenminimum ausgebildet.

Vorzugsweise ist vorgesehen, dass die ringförmigen Zonen und die Helixwindung auf einer gemeinsamen Seite ausgebildet und überlagert sind. Dies kann die Vorderseite oder aber auch die Rückseite sein. Durch eine derartige Ausgestaltung kann die Lage der beiden unterschiedlichen separaten Oberflächenstrukturen beziehungsweise Oberflächenprofile sehr genau erreicht werden.

Es kann auch vorgesehen sein, dass die ringförmigen Zonen auf einer Seite und die Helixwindung auf der anderen Seite ausgebildet sind. Dabei kann es vorgesehen sein, dass die ringförmigen Zonen auf der Vorderseite und die Helixwindung auf der Rückseite ausgebildet sind oder die Helixwindung auf der Vorderseite und die zumindest teilweise umlaufenden ringförmigen Zonen auf der Rückseite ausgebildet sind. Bei einer derartigen Ausgestaltung sind die individuellen separaten Oberflächenstrukturen beziehungsweise Oberflächenprofile in ihrer Kontur sehr genau herstellbar, so dass die Einzelstrukturen dann sehr präzise Abbildungseigenschaften ermöglichen.

Es kann auch vorgesehen sein, dass sowohl auf der einen Seite als auch auf der gegenüberliegenden anderen Seite ringförmige Zonen zumindest teilweise umlaufend ausgebildet sind. Ebenso kann zusätzlich oder anstatt dazu vorgesehen sein, dass die Helixwindung auf der einen Seite und auch auf der gegenüberliegenden anderen Seite ausgebildet ist. Diese beiden Helixwindungen sind dann so ausgebildet, dass sie in Summe die gesamte azimutale Brechkraft der Helixwindung als Beitrag zur gesamten Brechkraft der Augenlinse bilden. Im Vergleich zu einer Aufbringung der Helixwindung nur auf eine der Seiten wird bei einer derartigen Aufteilung somit die optische Wirkung bezüglich der Brechkraftverteilung auf die Seiten aufgeteilt.

Es ist vorgesehen, dass zumindest einige Zonen eine Hauptunterzone und eine Phasenunterzone aufweisen. Bei einer derartigen Ausgestaltung ist dann wieder eine, wie bereits aus dem Stand der Technik bekannte, stufenlose Topographie im Hinblick auf die optische Betrachtung und somit den Wellenfrontverlauf ausgebildet. Bezüglich der Bedeutung und technischen Auslegung einer Phasenunterzone darf auf das bereits oben Dargelegte hingewiesen werden.

Auch gelten bei einer derartigen Ausgestaltung dann vorzugsweise die bereits oben genannten Formeln für eine gemittelte Brechkraft und die Additionsbrechkraft im Besonderen.

Allerdings ist im Unterschied dazu dann hier die Gesamtbrechkraft der Augenlinse auch azimutal variierend und lässt sich durch nachfolgende Formel beschreiben:

$$F_{AV}(i, \alpha) = (1 - p(i, \alpha)) F_G(i, \alpha) + p(i, \alpha) F_S(i, \alpha) \qquad (3)$$

Dabei bedeuten i den Zonenindex und $\alpha$ den Azimutwinkel. Die Formel ist sowohl für bifokale Augenlinsen gültig als auch für zumindest trifokale Augenlinsen anwendbar bzw. modifizierbar. als auch Bei der nahegelegten allgemeinen Darstellung variiert somit die gemittelte Brechkraft der Augenlinse und insbesondere somit auch die Gesamtbrechkraft der Augenlinse abhängig vom Azimutwinkel $\alpha$ und insbesondere auch von der Nummer der Zone, wie sie in radialer Richtung angeordnet sind. In der genannten Formel 3 ist in dem Zusammenhang eine Variation der Brechkraft der Hauptunterzone als auch eine Variation der Brechkraft der Phasenunterzone als auch eine Variation des Flächenanteils der Hauptunterzone von diesem Azimutwinkel und dem Zonenindex angegeben. Es kann in dem Zusammenhang eine Variation aller dieser genannten Parameter von diesem Azimutwinkel und dem Ringindex beziehungsweise Zonenindex vorgesehen sein, andererseits können auch Ausführungen vorgesehen sein, bei denen beispielsweise die Hauptunterzone einer entsprechenden Variation unterliegt und die anderen Parameter keine derartige Variation umfassen. Entsprechend kann dies auch für eine Variation der Phasenunterzone ohne Variation der anderen Parameter vorgesehen sein.

Insbesondere setzt sich die azimutabhängige Gesamtbrechkraft der Augenlinse gemäß nachstehender Formel dar:

$$F_{ges}(\alpha, \rho) = F_{Sphäre}(\alpha) + F_{Cylinder}(\alpha, \rho) + F_{HOA}(\alpha, \rho) + F_{EDoF}(\alpha, \rho) \qquad (4)$$

mit

$\alpha$          = Azimutwinkel

$\rho$          = radiale Position auf dem optischen Teil

$F_{ges}$       = azimutabhängige Gesamtbrechkraft

$F_{Spähre}$     = sphärische Brechkraft

$F_{Cylinder}$    = azimutabhängiger zylindrischer Brechkraftanteil

$F_{HOA}=$     = azimutabhängiger Brechkraftanteil zur Korrektur von Aberration höherer Ordnung (bspw. Koma, Fünfblattfehler etc.)

$F_{EDoF}$      = azimutabhängiger Brechkraftanteil zur Beeinflussung der Schärfentiefe

Vorzugsweise beträgt ein Flächenanteil p einer Hauptunterzone an der Gesamtfläche einer Zone ≥ 80 %.

Insbesondere gilt: $F_{ges}(\alpha, \rho) = F_{AV}(i, \alpha)$

Die multifokale Linse ist als Diffraktionslinse ausgebildet. Bezüglich einer Ringdichte rm ($\alpha$), die somit auch einen Normierungsradius darstellt und eine Anzahl von zumindest teilweise umlaufenden ringförmigen Zonen pro vorgegebenem Radiusintervall beschreibt und somit quasi eine Gitterkonstante der Zonen deklariert, trägt diese ebenfalls zu einer weiteren Beschreibung der multifokalen Linse bei. Entsprechendes gilt dann auch für einen Phasenhub Φ, der quasi in radialer Richtung den optisch wirksamen Versatz zwischen zwei benachbarten Zonen beschreibt. Der Phasenhub bestimmt somit auch die Energieaufteilung zwischen den Beugungsordnungen. Die Ringdichte bestimmt den Wert der diffraktiven Additionsbrechkraft, was bedeutet, dass sie den Winkelabstand der Beugungsordnungen bestimmt.

Insbesondere in Kombination der oben genannten Formel für die Gesamtbrechkraft, die bezüglich ihrer Verteilung vom Azimutwinkel, von der radialen Position auf der jeweiligen optischen Seite und dem Zonenindex abhängt, kann insbesondere in Kombination mit der oben genannten lokalen azimutabhängigen Ringdichte, des Phasenhubs, gegebenenfalls periodisch alternierenden Parametern, die insbesondere für trifokale und noch höher fokale Ausgestaltungen erforderlich sind, eine vollständige allgemeine Beschreibung der Brechkraftverteilung der Augenlinse erfolgen. Auch dies lässt sich mittels bekannter Methoden, wie beispielsweise Raytracing, die Wellenfront und/oder die Topographie einer Ausgestaltung eines optischen Teils der Augenlinse bestimmen.

Es ist vorgesehen, dass eine durch die Hauptunterzone zur Gesamtbrechkraft der Augenlinse beitragende Hauptunterzonen-Brechkraft in azimutaler Richtung um die Hauptachse zumindest einmal variiert. Zusätzlich oder anstatt dazu ist es vorgesehen, dass eine durch die Phasenunterzone zur Gesamtbrechkraft der Augenlinse beitragende Phasenunterzonen-Brechkraft in azimutaler Richtung um die Hauptachse zumindest einmal variiert. Dadurch lässt sich die Brechkraftverteilung derartig spezifischer Linsen mit einer Hauptunterzone und einer Phasenunterzone ganz individuell und präzise beeinflussen, so dass bei mehreren Hauptbrechkräften der Augenlinse und somit bei mehreren Foki der Augenlinse auch deren Schärfentiefe individuell erweitern lässt.

Es ist vorgesehen, dass ein Phasenhub $\Phi$ zwischen zwei benachbarten Zonen im Intervall $< \lambda$ liegt. $\lambda$ betrifft hier die Designwellenlänge, die vorzugsweise in dem bereits oben genannten Intervallbereich liegt.

Durch derartig kleine Phasenhübe sind Ausgestaltungen realisiert, die sich wesentlich auch von gestuften Linsen mit Fresnelzonen unterscheiden, da bei derartigen Fresnellinsen der Phasenhub üblicherweise ein Vielfaches der Designwellenlänge beträgt. Gerade bei Ausgestaltungen der erfindungsgemäßen multifokalen Augenlinse, bei der die Zonen und die Zonenübergänge im Sinne der oben genannten Definition stufenlos sind, ergibt sich gegenüber Fresnellinsen eine völlig unterschiedliche Ausgestaltung.

Vorzugsweise ist vorgesehen, dass der Phasenhub zumindest bei einer Zone, insbesondere bei den von der optischen Hauptachse radial bemessenen, nach außen folgenden inneren Zonen, in azimutaler Richtung um die Hauptachse konstant ist. Die Hauptbrechkräfte - also die Brechkräfte, die in der Intensitätsverteilung des Brechkraftspektrums deutlich und insbesondere um das mehrfache gegenüber anderen Spitzen erhöht sind - und somit die Foki erfahren hier praktisch keine Längenausdehnung in ihren Intensitätsverteilungen.

In einer vorteilhaften Ausführung ist vorgesehen, dass der Phasenhub zumindest bei einer Zone, insbesondere bei den von der optischen Hauptachse radial nach außen gezählten ersten Zonen, in azimutaler Richtung um die Hauptachse variiert ist. Durch eine Variation des Phasenhubs in azimutaler Richtung können Breiten- und somit Längenausdehnungen individueller Hauptbrechkräfte und somit individueller Foki in den Intensitätsverteilungen erreicht werden. Dadurch können die individuellen Foki der Augenlinse bezüglich ihrer Intensitätsverteilung ganz präzise und individuell gewichtet werden.

Es kann vorgesehen sein, dass der Phasenhub stufenlos variiert ist.

Ebenso ist die Variation des Phasenhubs auch dahingehend möglich, dass zumindest einmal in dem vollständigen Umlauf um die optische Hauptachse eine diskrete Phasenhubstufe ausgebildet ist.

Mit einem Phasenhub wird diejenige Einstellung erreicht, wie viel Licht zwischen der nullten und ersten Beugungsordnung aufgeteilt wird.

Es ist vorgesehen, dass der Phasenhub über zumindest ein erstes Teilazimutintervall $= 0$ ist und über zumindest ein zweites Teilazimutintervall $\neq 0$ ist. Bei einer derartigen zumindest einmaligen diskreten oder kontinuierlichen Phasenhubvariation in einem Umlauf um die optische Achse ist dann insbesondere vorgesehen, dass abhängig von dem Verhältnis zwischen dem ersten und dem zweiten Teilazimutintervall ein Verhältnis zwischen einer Fernbrechkraft und einer Nahbrechkraft der Augenlinse veränderbar ist. Dies bedeutet, dass abhängig davon, über welche Winkelintervalle sich die beiden Teilazimutintervalle jeweils erstrecken, ein Quotient erzeugt wird, wobei abhängig davon dann die Aufteilung zwischen den Intensitäten einer Fernbrechkraft und einer Nahbrechkraft, die Hauptbrechkräfte der Augenlinse darstellen, gebildet ist.

Es kann auch vorgesehen sein, dass der Phasenhub in radialer Richtung variiert, so dass beispielsweise zwischen zwei radial aufeinander folgenden Zonen ein erster Phasenhub ausgebildet ist, und zwischen zwei weiteren radial aufeinander folgenden Zonen ein dazu unterschiedlicher zweite Phasenhub ausgebildet ist. Dies kann bei Ausführungen vorgesehen sein, bei denen der Phasenhub zwischen zwei Zonen auf azimutal variiert als auch bei Ausführungen, bei denen eine derartige azimutale Variation nicht ausgebildet ist.

Insbesondere ist vorgesehen, dass die Helixwindung einmal um die Hauptachse umlaufend ausgebildet ist und zwischen einem Windungsanfang und einem Windungsende ein sprungartiger Übergang ausgebildet ist. Durch einen derartigen möglichst diskreten Übergang, der in azimutaler Richtung um die optische Hauptachse über einen möglichst kleinen Winkelbereich, insbesondere $< 20°$, insbesondere $< 10°$, ausgebildet ist, wird die sich verändernde, insbesondere kontinuierlich verändernde, insbesondere vom Windungsanfang zum Windungsende kontinuierlich erhöhende, Brechkraftverteilung in azimutaler Richtung begünstigt. Durch einen möglichst azimutal kleinen Übergang wird die optische Abbildungseigenschaft der Augenlinse verbessert.

Vorzugsweise ist vorgesehen, dass die Augenlinse zusätzlich zu den ringförmigen Zonen und der Helixwindung ein torisch brechendes Oberflächenprofil aufweist, und diese torisch brechende Oberflächenform auf zumindest einer Seite des optischen Teils ausgebildet ist. Durch Ergänzung eines torisch brechenden Oberflächenprofils beziehungsweise einer Oberflächenform kann auch eine Astigmatismuskorrektur erfolgen. Auch hier kann die torisch brechende Oberflächenform auf einer oder auf zwei Seiten verteilt ausgebildet sein.

Insbesondere ist vorgesehen, dass der Übergang in azimutaler Richtung gegenüber einem flachen Hauptmeridian der torisch brechenden Oberflächenform versetzt ist. Insbesondere ist der sprungartige Übergang in azimutaler Lage um die optische Hauptachse in einem azimutalen Winkel zwischen 20° und 70°, insbesondere zwischen 40° und 50° versetzt. Es kann auch vorgesehen sein, dass der sprungartige Übergang in azimutaler Lage um die optische Hauptachse in einem azimutalen Winkel zwischen 200° und 250°, insbesondere zwischen 220° und 230°, zu einem flachen Hauptmeridian der torisch brechenden Oberfläche ausgebildet ist. Vorzugsweise ist der azimutale Lageverschub zwischen dem sprungartigen Übergang und dem flachen Hauptmeridian zwischen $(1/4\ \pi)$ +/- $(1/8\ \pi)$ oder $(5/4\ \pi)$ +/- $(1/8\ \pi)$. Auf diese spezifischen Lagen kann die Dicke der Linse in Richtung der optischen Hauptachse deutlich reduziert werden und somit diesbezüglich minimiert werden. Dadurch wird die Kleinschnitttauglichkeit erhöht, was bedeutet, dass die Linse sehr klein gefaltet werden kann und durch einen möglichst kleinen Schnitt im Auge als Intraokularlinse in das Auge eingeführt werden kann.

Insbesondere bezieht sich dieser azimutale Winkelversatz auf eine azimutale Mitte des sich azimutal über eine endliche Breite ausbildenden Übergangs, insbesondere eine Mitte bei einer Projektion des Übergangs in eine Ebene senkrecht zur optischen Hauptachse. Vorzugsweise ist vorgesehen, dass die Augenlinse zumindest bifokal ausgebildet ist und somit zumindest zwei Hauptbrechkräfte aufweist, die eine Fernbrechkraft und eine Nahbrechkraft sind. Die Augenlinse weist somit $n \geq 2$ Foki auf und die Helixwindung ist bei einer Ausführung so ausgebildet, dass maximal $n - 1$ Foki durch die Helixwindung in der Schärfentiefe vergrößert sind. Auch dadurch ist eine individuelle Gestaltung einer Augenlinse bezüglich ihrer Charakterisierung der Abbildungseigenschaften in den einzelnen Foki erreicht.

Eine weitere vorteilhafte Ausgestaltung ist darin zu sehen, dass ein Radius zumindest einer Zone in azimutaler Richtung um die optische Hauptachse zumindest einmal variiert ist. Dadurch können auch Zonen gebildet werden, die nicht streng über ihre gesamte azimutale Länge einen Radius und somit eine Kreisbogenbahn aufweisen, sondern die auch beispielsweise als polygonartige Teilringe oder dann auch vollständige Polygonringe ausgebildet werden können.

Vorzugsweise ist vorgesehen, dass Radien von mehreren Zonen azimutal zumindest einmal variierend sind und die Azimutlage der Variation bei den mehreren Zonen gleich ist. Dadurch werden quasi mehrere in radialer Richtung aufeinanderfolgende Zonen geschaffen, die im Hinblick auf ihre azimutale Konturengestaltung in der Form gleich ausgebildet sind.

Auch dadurch lassen sich individuelle Ausgestaltungen von einzelnen Hauptbrechkräften und somit Foki der multifokalen Augenlinse erreichen.

Vorzugsweise ist vorgesehen, dass auf einer Seite des optischen Teils in azimutaler Richtung um die Hauptachse ein erster kuchenstückförmiger Oberflächensektor und zumindest ein zweiter kuchenstückförmiger Oberflächensektor ausgebildet ist, die in azimutaler Richtung überlappungsfrei ausgebildet sind. Bevorzugt ist vorgesehen, dass die Zonenzahl und/oder die Flächengrößen der Zonen in den zumindest zwei Oberflächensektoren unterschiedlich sind. Auch dadurch lässt sich eine Topographie zumindest einer Seite des optischen Teils ganz individuell gestalten und somit optische Abbildungseigenschaften der multifokalen Linse gezielt beeinflussen, so dass auch hier insbesondere die Schärfentiefe in zumindest einem Fokus der Augenlinse individuell beeinflussbar ist.

Ein weiterer Aspekt der Erfindung betrifft eine multifokale Intraokularlinse mit einem optischen Teil, durch welchen die optischen Abbildungseigenschaften der Intraokularlinse charakterisiert sind. Der optische Teil weist eine in Richtung einer optischen Hauptachse der Intraokularlinse betrachtet erste optische Seite und eine gegenüberliegende zweite optische Seite auf. Der optische Teil umfasst zumindest auf einer Seite eine Mehrzahl von um die optische Hauptachse zumindest teilweise umlaufende, ringförmige optische Zonen, wobei Zonen jeweils zumindest eine Hauptunterzone aufweisen und zwischen zwei benachbarten Zonen ein Phasenhub ausgebildet ist. Die Zonen sind in radialer Richtung benachbart ausgebildet und aneinander angrenzend angeordnet.

Ein wesentlicher Gedanke des weiteren Aspekts der Erfindung ist darin zu sehen, dass eine Gesamtbrechkraft der Intraokularlinse in azimutaler Richtung bei einem Umlauf um die Hauptachse ohne ein Zwischenminimum ansteigend variiert. Dies bedeutet, dass bei einem einzigen Umlauf um die optische Hauptachse ausgehend von einem ersten Wert der Gesamtbrechkraft an einem Anfang auf einen zweiten Wert der Gesamtbrechkraft an einem Ende dieses vollständigen Umlaufs ansteigt und dazwischen kein Minimum auftritt. Eine derartige zwischenminimumlose Ausgestaltung ermöglicht ebenfalls eine ganz individuelle Vergrößerung einer Schärfentiefe der multifokalen Linse in zumindest einem gezielten Fokus und somit in zumindest einer spezifischen Hauptbrechkraft. Insbesondere bei multifokalen Intraokularlinsen , bei denen die angesprochenen, zumindest teilweise um die optische Hauptachse umlaufenden ringförmigen Zonen eine Hauptunterzone und eine Phasenunterzone aufweisen, sind auch die zweiten und dritten Aspekte der Erfindung vorteilhaft.

Ausführungen des ersten Aspekts der Erfindung sind als vorteilhafte Ausführungen des weiteren Aspekts der Erfindung anzusehen.

Gerade bei Ausgestaltungen einer Augenlinse als Intraokularlinse ist diese dann im Auge mit einem Immersionsmedium, dem Kammerwasser, mit seinem Brechungsindex $n_{med}$ von 1,336 , angeordnet.

Bei der Ausgestaltung einer multifokalen Augenlinse, bei welcher Zonen aus einer Hauptunterzone und einer Phasenunterzone aufgebaut sind, sind die Hauptunterzonen ebenfalls um die optische Hauptachse zumindest teilweise umlaufende, ringförmige Gebilde. Ebenso sind die Phasenunterzonen dann zumindest teilweise um die optische Hauptachse umlaufende, ringförmige Gebilde. Eine Hauptunterzone grenzt in radialer Richtung direkt an eine Phasenunterzone einer betrachteten Zone an. Gemeinsam bildet die zumindest eine Hauptunterzone und die zumindest eine Phasenunterzone einer derartigen Zone die gesamte radiale Ausdehnung der betrachteten Zone.

Weitere Merkmale der Erfindung ergeben sich aus den Ansprüchen, den Figuren und der Figurenbeschreibung. Die vorstehend in der Beschreibung genannten Merkmale und Merkmalskombinationen, sowie die nachfolgend in der Figurenbeschreibung genannten und/oder in den Figuren alleine gezeigten Merkmale und Merkmalskombinationen sind nicht nur in der jeweils angegebenen Kombination, sondern auch in anderen Kombinationen oder in Alleinstellung verwendbar, ohne den Rahmen der Erfindung zu verlassen. Es sind somit auch Ausführungen von der Erfindung als umfasst und offenbart anzusehen, die in den Figuren nicht explizit gezeigt und erläutert sind, jedoch durch separierte Merkmalskombinationen aus den erläuterten Ausführungen hervorgehen und erzeugbar sind.

Die in den Unterlagen angegebenen konkreten Werte von Parametern und Angaben zu Verhältnissen von Parametern bzw. Parameterwerten zur Definition von Ausführungsbeispielen der Augenlinse sind auch im Rahmen von Abweichungen, beispielsweise aufgrund von Messfehlern, Systemfehlern, DIN-Toleranzen etc. als vom Rahmen der Erfindung mit umfasst anzusehen, wodurch auch Erläuterungen, die sich auf im Wesentlichen entsprechende Werte und Angaben beziehen darunter zu verstehen sind.

Kurze Beschreibung der Zeichnungen

Ausführungsbeispiele der Erfindung werden nachfolgend anhand schematischer Zeichnungen näher erläutert. Es zeigen:

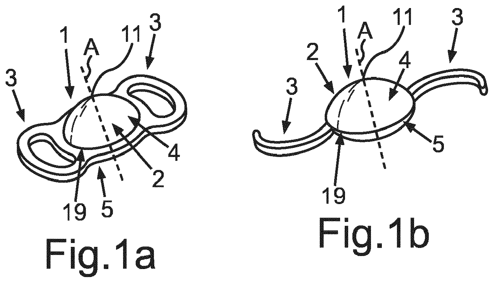

Fig. 1a        eine perspektivische Darstellung eines ersten Ausführungsbeispiels einer erfindungsgemäßen Augenlinse;

Fig. 1b        eine perspektivische Darstellung eines weiteren Ausführungsbeispiels einer erfindungsgemäßen Augenlinse;

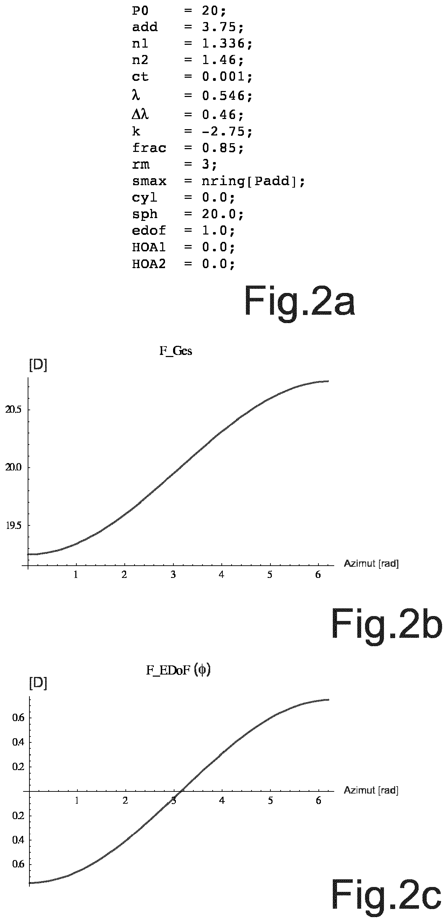

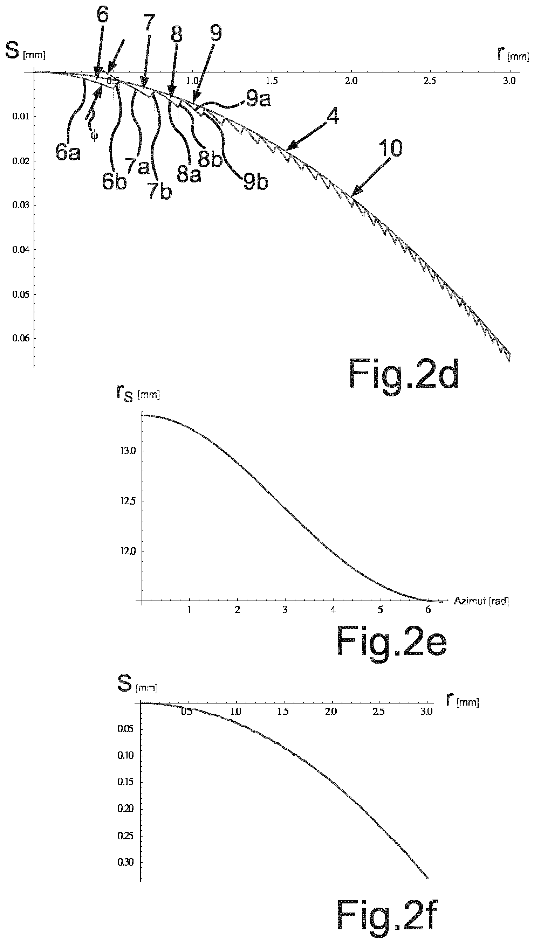

Fig. 2a bis 2h        eine Darstellung eines Ausführungsbeispiels einer erfindungsgemäßen Augenlinse mit spezifischen Parameterwerten und Diagrammen, in denen Parameter abhängig vom Azimut dargestellt sind, sowie eine schematische dreidimensionale Oberflächendarstellung einer Seite eines optischen Teils der Augenlinse;

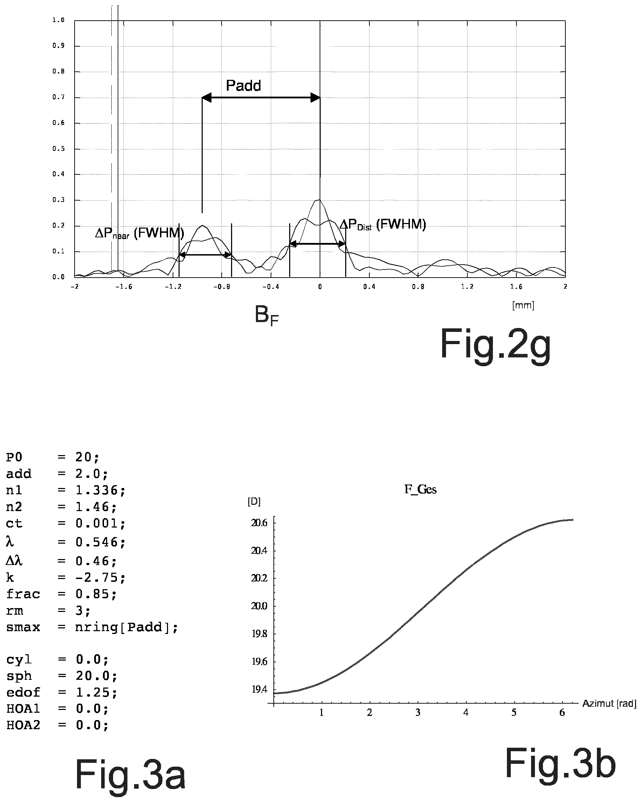

Fig. 3a bis 3g        eine Darstellung eines zweiten Ausführungsbeispiels einer erfindungsgemäßen Augenlinse mitspezifischen Parameterwerten und Diagrammen, in denen Parameter in Abhängigkeit vom Azimut beziehungsweise dem Radius dargestellt sind, und eine schematische Darstellung einer dreidimensionalen Oberfläche einer Seite eines optischen Teils der Augenlinse dieses Ausführungsbeispiels;

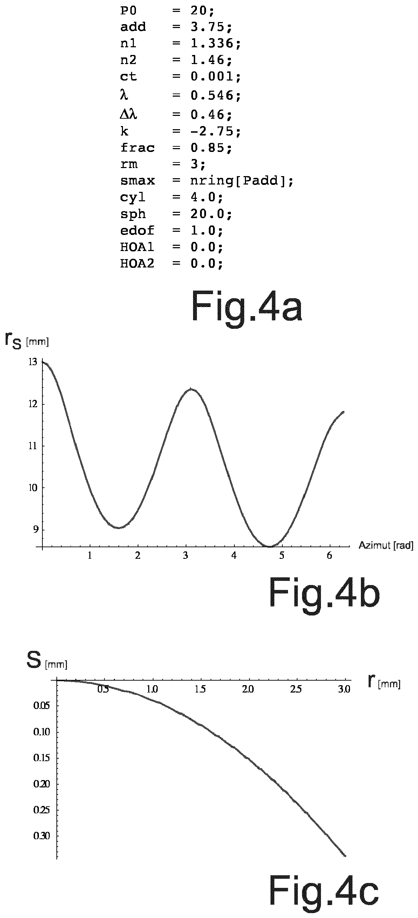

Fig. 4a bis 4e        eine Darstellung eines weiteren Ausführungsbeispiels einer erfindungsgemäßen Augenlinse mit spezifischen Parameterwerten und einzelnen Parametern in Abhängigkeit des Azimuts sowie eine schematische Darstellung einer dreidimensionalen Oberfläche einer Seite eines optischen Teils der Augenlinse;

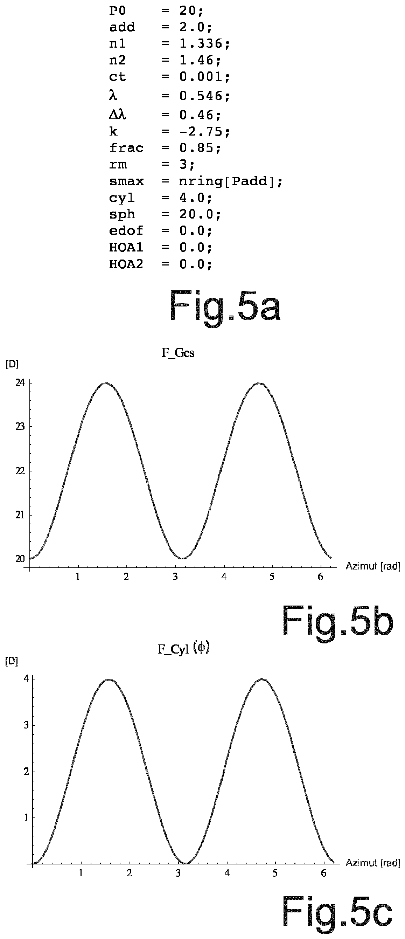

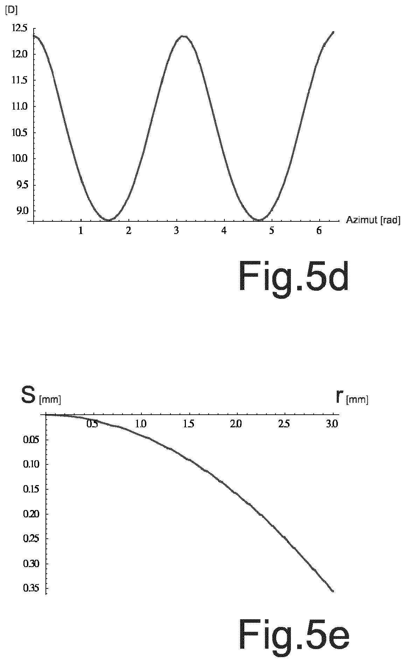

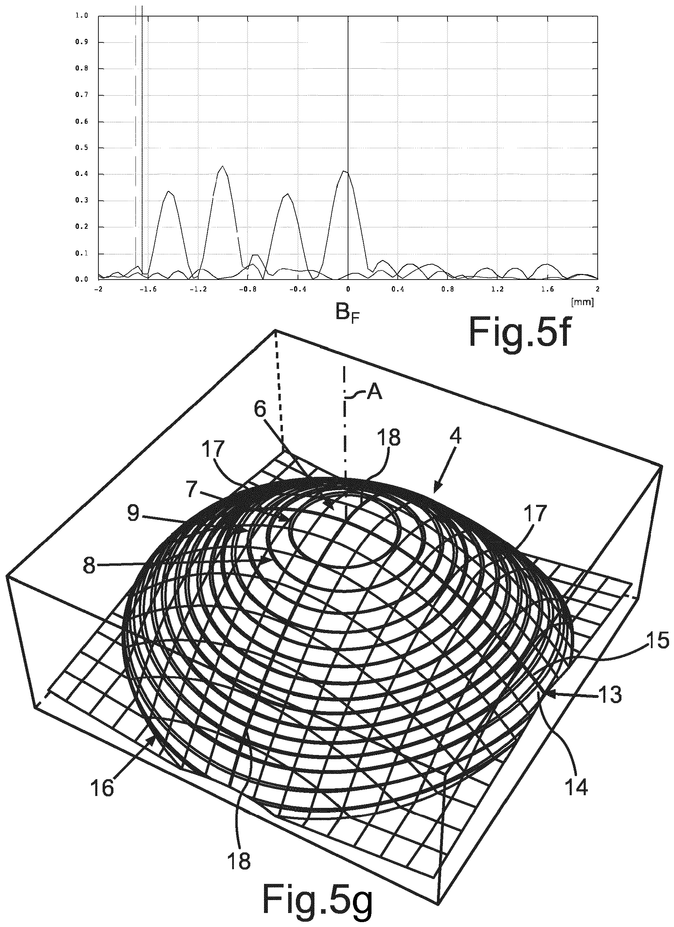

Fig. 5a bis 5g        ein weiteres Ausführungsbeispiel einer erfindungsgemäßen Augenlinse in spezifischen Parameterwerten sowie Diagrammen, in denen Parameterwerte in Abhängigkeit des Azimuts beziehungsweise des Radius dargestellt sind, und eine schematische Darstellung eines Oberflächenprofils einer Seite eines optischen Teils der Augenlinse;

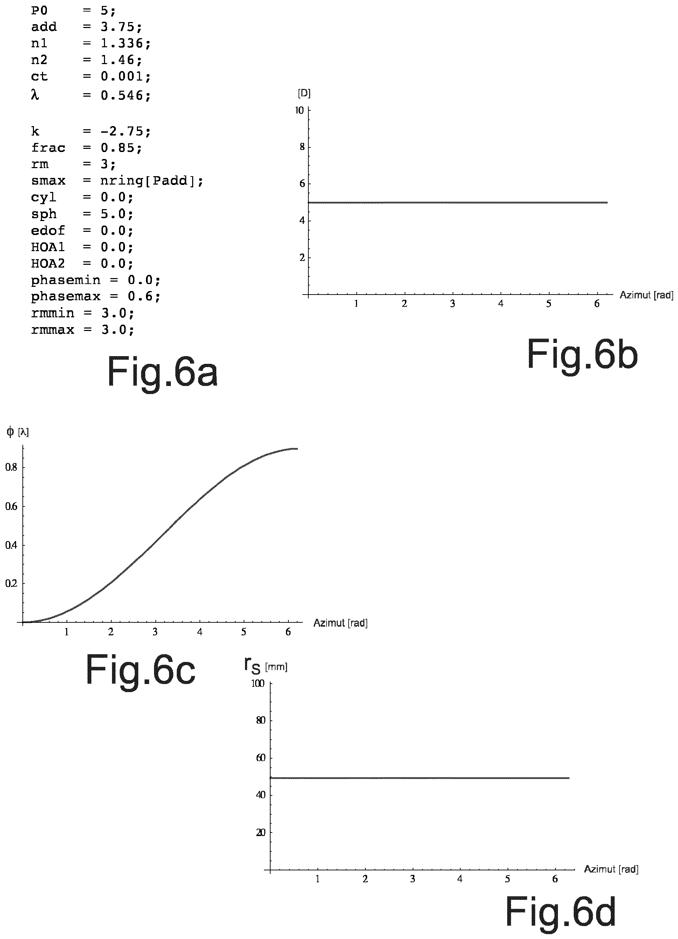

Fig. 6a bis 6g        eine Darstellung eines weiteren Ausführungsbeispiels mit spezifischen Parameterwerten und Diagrammen, in denen Parameterwerte in Abhängigkeit vom Azimut beziehungsweise vom Radius dargestellt sind, sowie eine schematische Darstellung einer dreidimensionalen Oberflächenform einer Seite eines optischen Teils der Augenlinse;

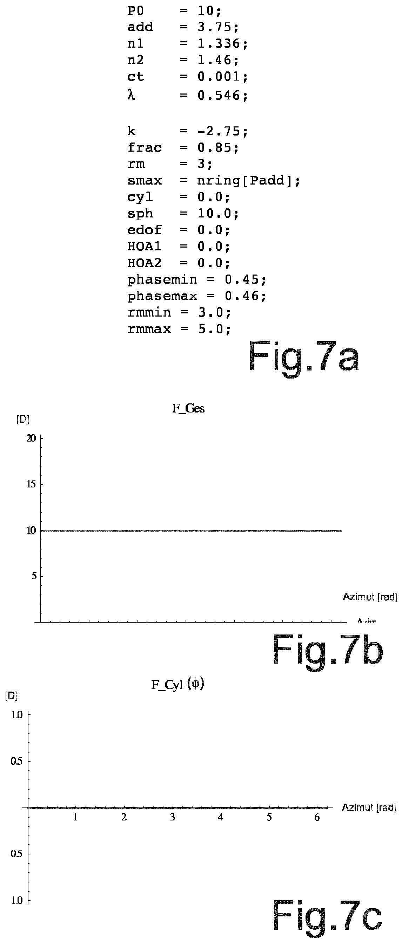

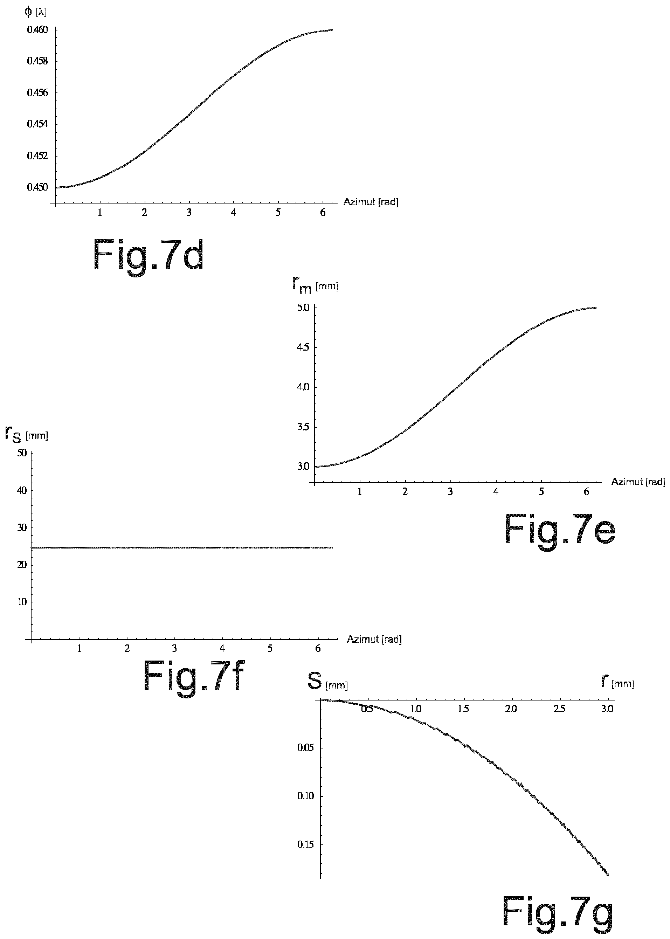

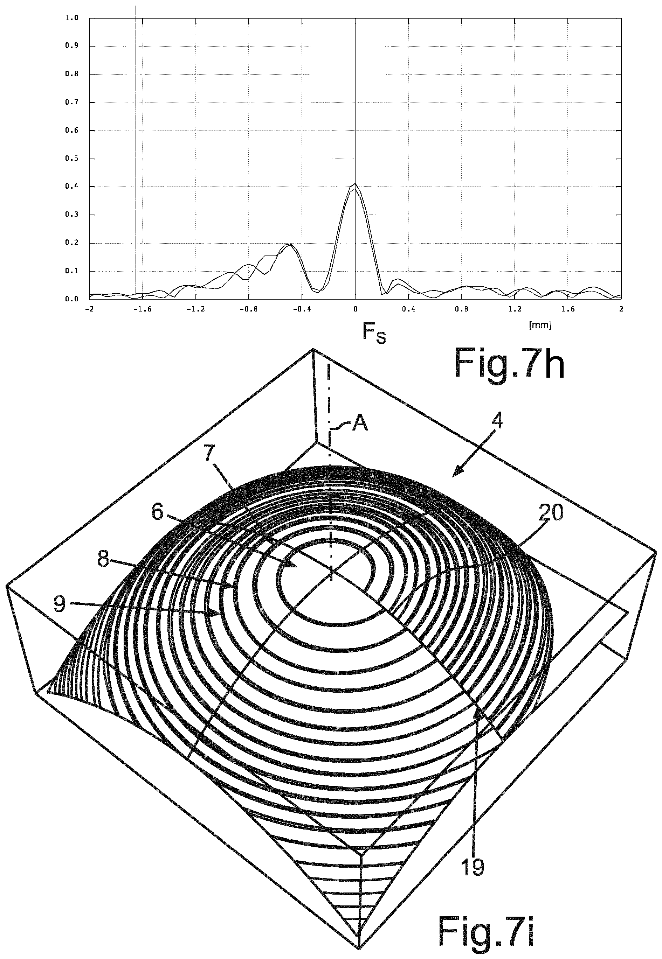

Fig. 7a bis 7i — eine Darstellung eines weiteren Ausführungsbeispiels einer erfindungsgemäßen Augenlinse mit spezifischen Parameterwerten und Diagrammen, in denen Parameter in Abhängigkeit vom Azimut und dem Radius dargestellt sind, sowie eine schematische Darstellung einer dreidimensionalen Oberflächenform einer Seite eines optischen Teils der Augenlinse;

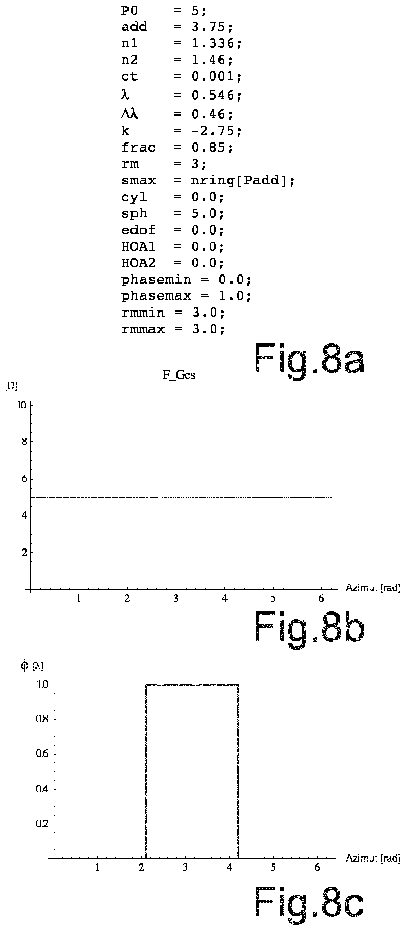

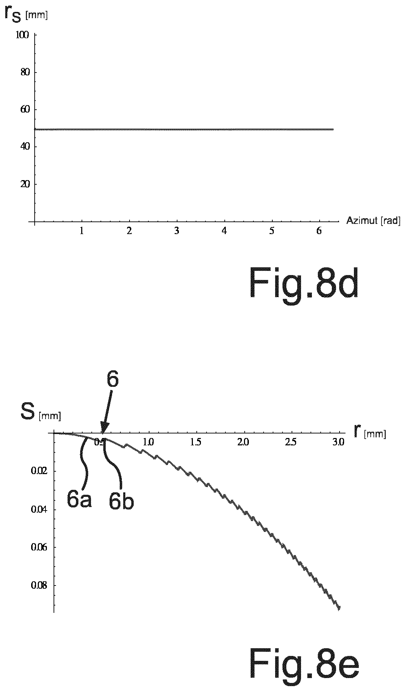

Fig. 8a bis 8f — ein weiteres Ausführungsbeispiel einer erfindungsgemäßen Augenlinse mit spezifischen Parameterwerten und Diagrammen, in denen Parameter in Abhängigkeit vom Azimut und dem Radius dargestellt sind, sowie eine schematische Darstellung einer dreidimensionalen Oberflächenform einer Seite eines optischen Teils der Augenlinse;

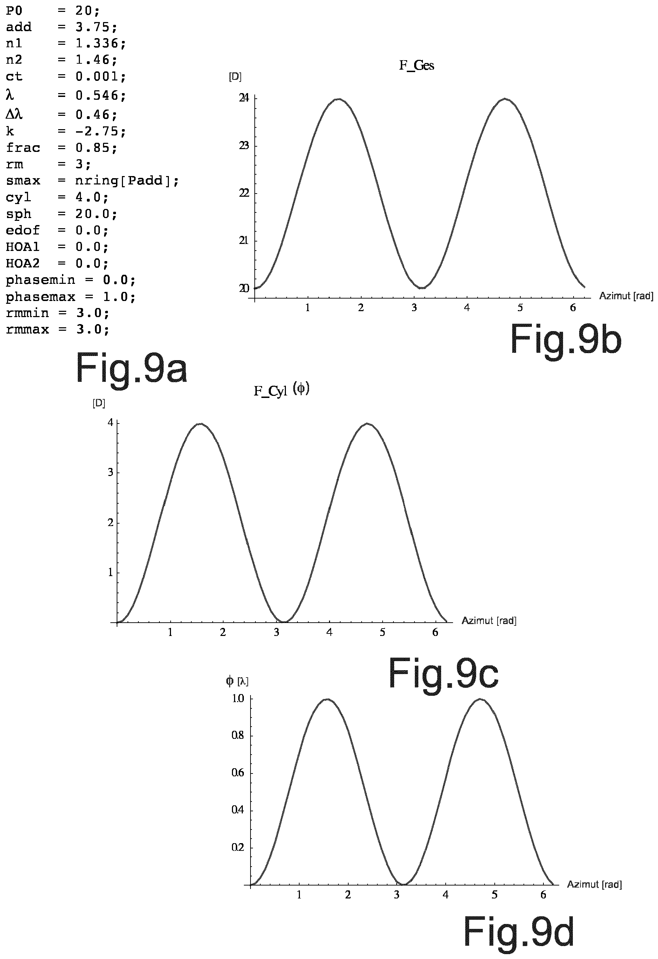

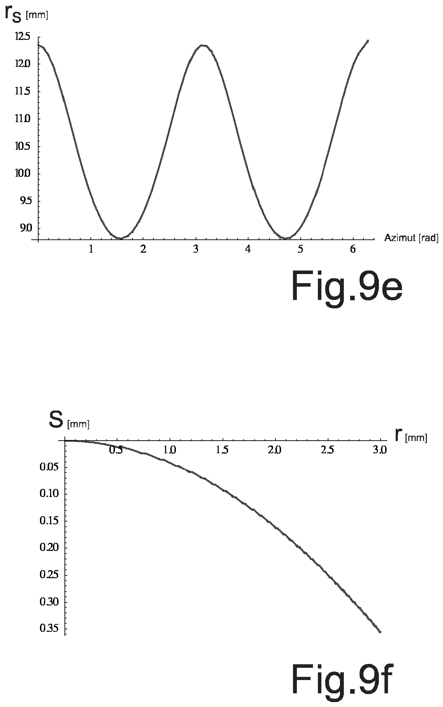

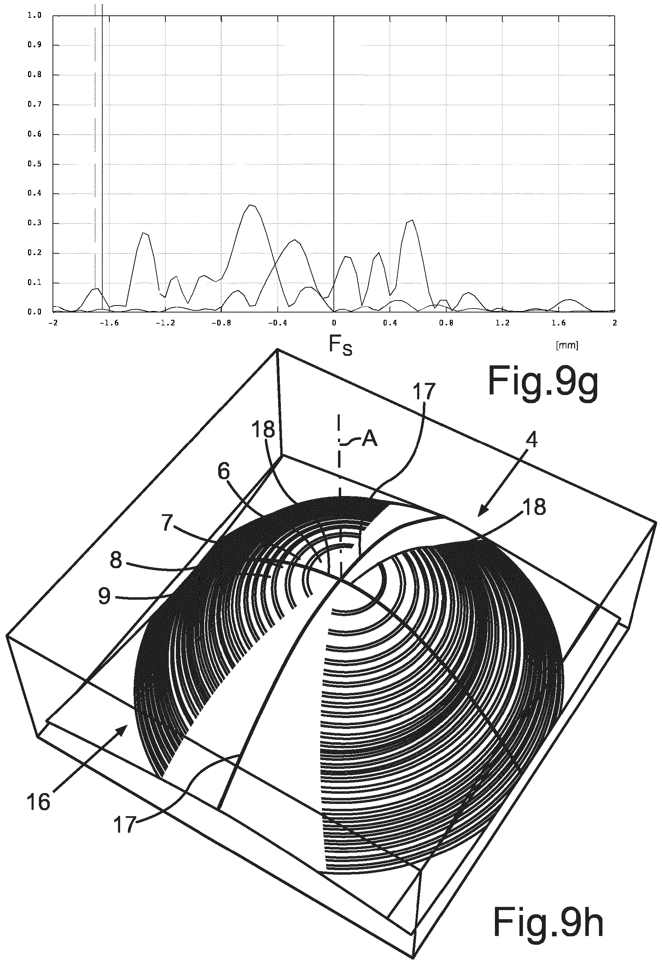

Fig. 9a bis 9h — eine Darstellung eines weiteren Ausführungsbeispiels einer erfindungsgemäßen Augenlinse mit spezifischen Parameterwerten und Diagrammen, in denen Parameter in Abhängigkeit vom Azimut beziehungsweise dem Radius dargestellt sind, sowie eine schematische Darstellung einer dreidimensionalen Oberflächenform einer Seite eines optischen Teils der Augenlinse;

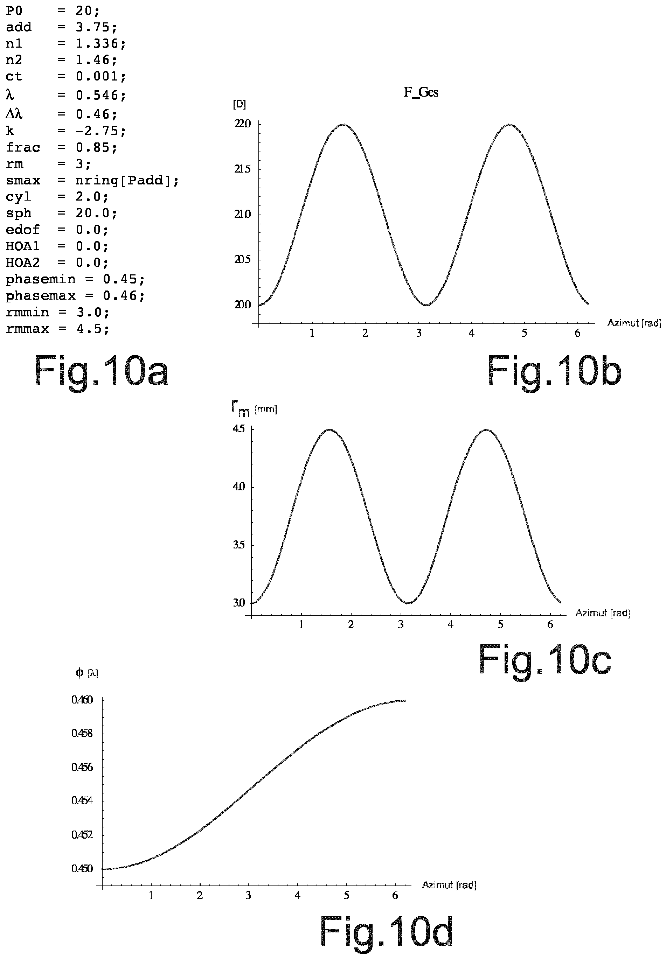

Fig. 10a bis 10g — ein weiteres Ausführungsbeispiel einer erfindungsgemäßen Augenlinse mit spezifischen Parameterwerten und Diagrammen, in denen Parameter in Abhängigkeit vom Azimut beziehungsweise dem Radius dargestellt sind, und einer schematischen Darstellung einer dreidimensionalen Oberflächenform einer Seite eines optischen Teils dieser Augenlinse;

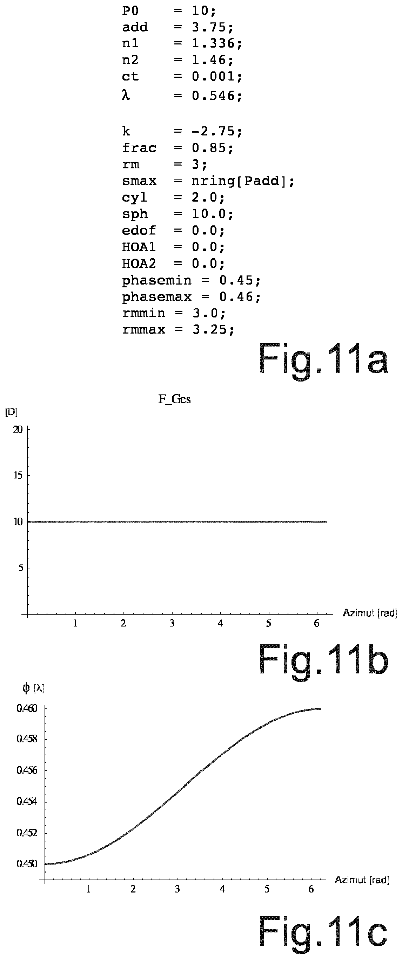

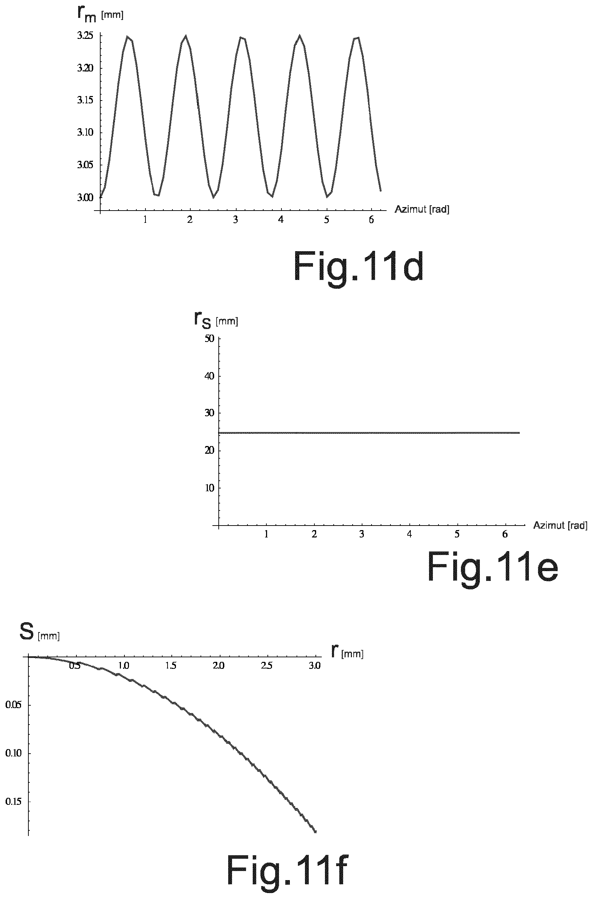

Fig. 11a bis 11h — eine Darstellung eines weiteren Ausführungsbeispiels einer erfindungsgemäßen Augenlinse mit spezifischen Parameterwerten und Diagrammen, in denen Parameter in Abhängigkeit vom Azimut beziehungsweise vom Radius dargestellt sind, und eine schematische Darstellung einer dreidimensionalen Oberflächenform einer Seite eines optischen Teils der Augenlinse;

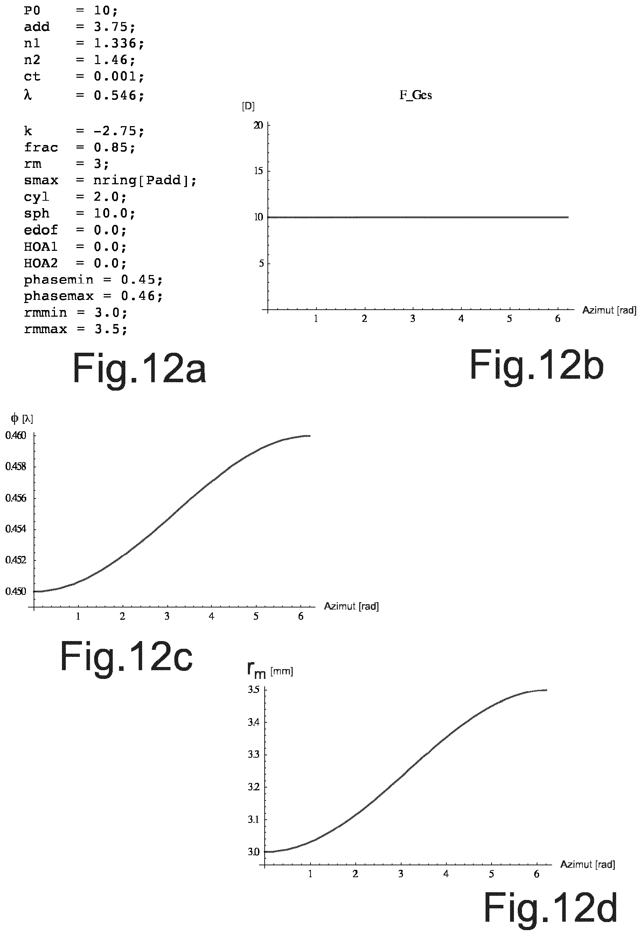

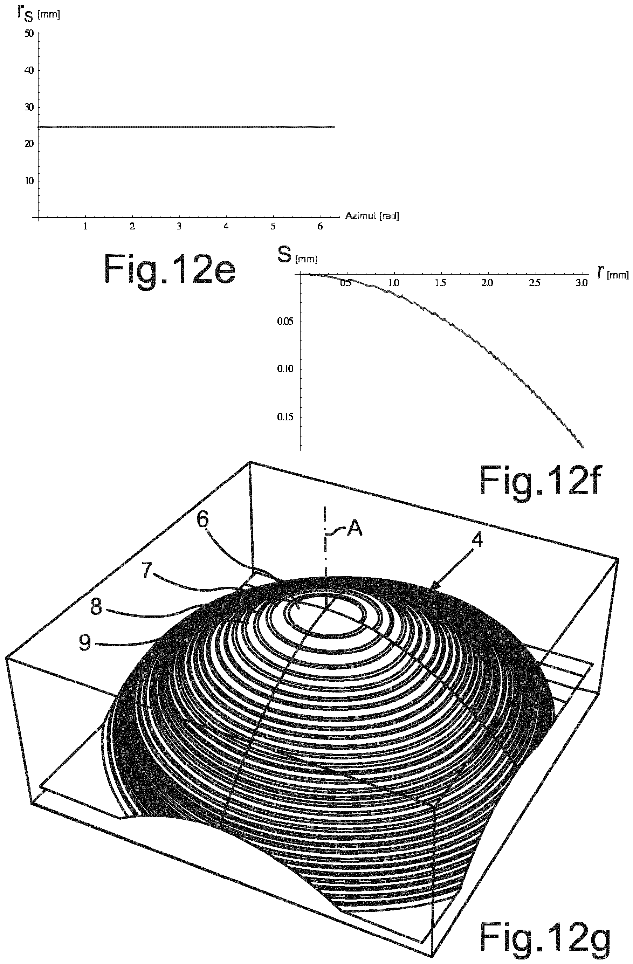

Fig. 12a bis 12g — eine Darstellung eines weiteren Ausführungsbeispiels einer erfindungsgemäßen Augenlinse mit spezifischen Parameterwerten und Diagrammen, in denen Parameter in Abhängigkeit vom Azimut beziehungsweise vom Radius dargestellt sind, und eine schematische Darstellung einer dreidimensionalen Oberfläche einer Seite eines optischen Teils dieser Augenlinse;

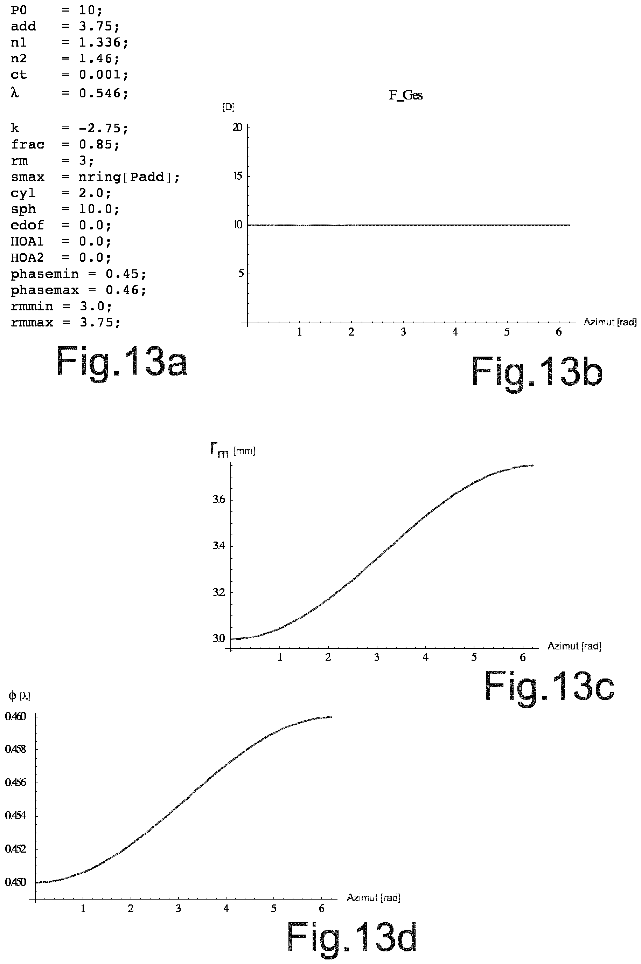

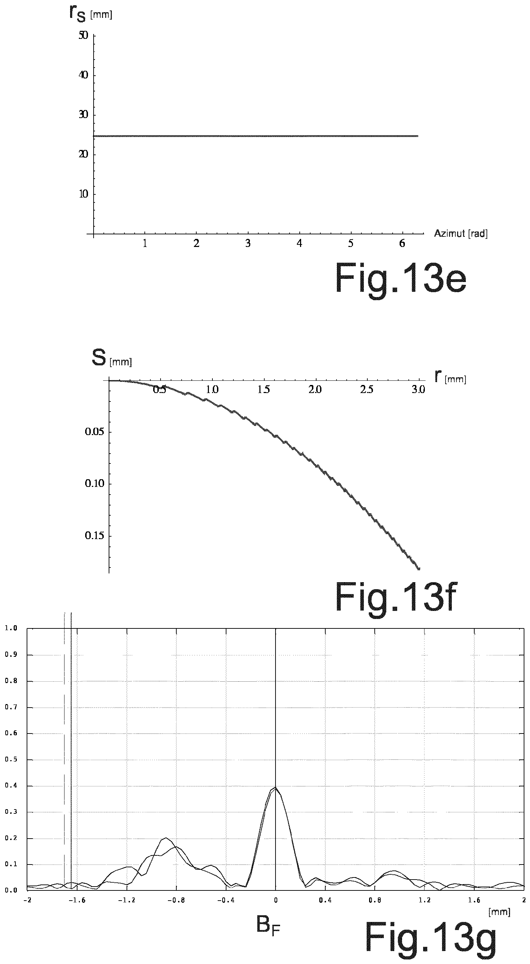

Fig. 13a bis 13g — eine Darstellung eines weiteren Ausführungsbeispiels einer erfindungsgemäßen Augenlinse mit spezifischen Parameterwerten und Diagrammen, in denen Parameter in Abhängigkeit vom Azimut beziehungsweise Radius dargestellt sind; und

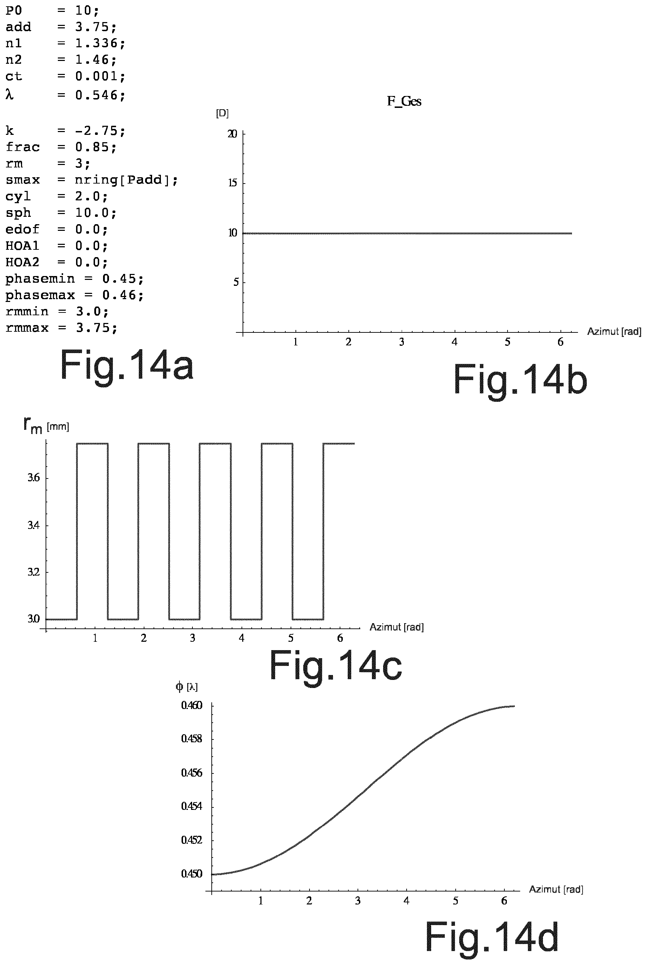

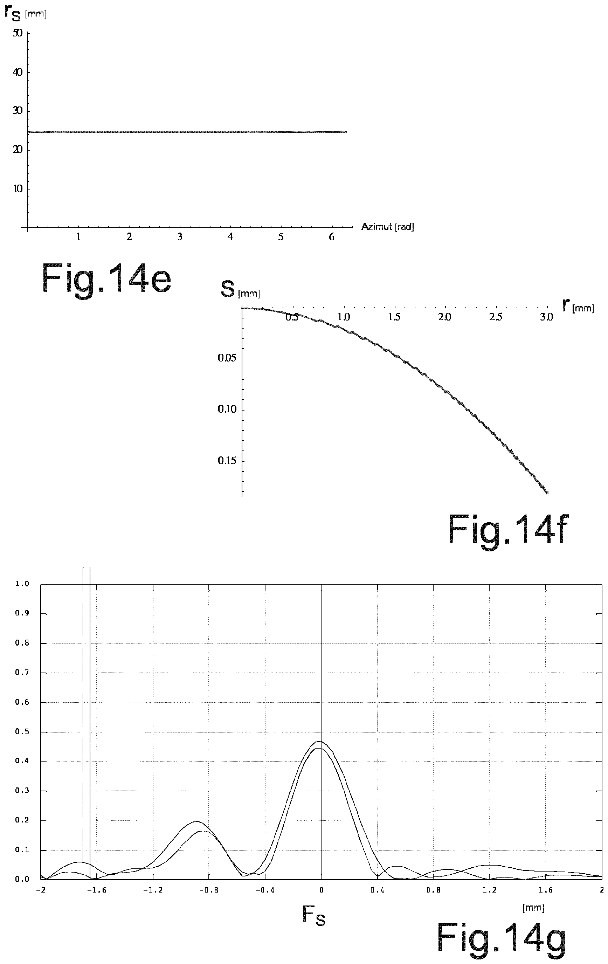

Fig. 14a bis 14h — ein weiteres Ausführungsbeispiel einer erfindungsgemäßen Augenlinse mit spezifischen Parameterwerten und Diagrammen, in denen Parameter in Abhängigkeit vom Azimut beziehungsweise vom Radius dargestellt sind, sowie eine schematische Darstellung einer dreidimensionalen Oberflächenform eines optischen Teils dieser Augenlinse.

In den Figuren werden gleiche oder funktionsgleiche Elemente mit den gleichen Bezugszeichen versehen.

In Fig. 1a ist in einer perspektivischen Darstellung ein erstes Ausführungsbeispiel einer Augenlinse 1 gezeigt, die eine Intraokularlinse ist. Die Augenlinse 1 umfasst einen optischen Teil 2 und daran anschließend eine Haptik 3. Die Augenlinse 1 ist faltbar und über einen Kleinschnitt in ein Auge einführbar. Der optische Teil 2, der wesentlich für die optische Abbildungseigenschaft der Augenlinse 1 ist, umfasst eine optische Hauptachse A. Der optische Teil 2 weist darüber hinaus in Richtung dieser optischen Hauptachse A betrachtet eine erste optische Fläche bzw. Seite 4, die eine Vorderseite sein kann, und gegenüberliegend eine zweite optische Fläche bzw. Seite 5, die eine Rückseite sein kann, auf. Die beispielhafte Vorderseite 4 ist im implantierten Zustand der Augenlinse 1 im Auge der Hornhaut zugewandt, wohingegen die Rückseite dieser Hornhaut abgewandt ist.

In Fig. 1b ist in einer perspektivischen Darstellung ein weiteres Ausführungsbeispiel einer als Intraokularlinse ausgebildeten Augenlinse 1 gezeigt. Sie unterscheidet sich von der Ausführung in Fig. 1a durch die unterschiedliche Haptik 3. Mittels der Haptik 3 ist die Augenlinse 1 im Auge gehalten.

Grundsätzlich können auch anderweitig geformte und ausgestaltete Haptiken 3 vorgesehen sein.

In Fig. 2a ist ein Parametersatz für ein Ausführungsbeispiel einer erfindungsgemäßen multifokalen Augenlinse angegeben. Dabei bezeichnet $P_0$ eine Grundbrechkraft der Augenlinse in Dioptrie, "add" eine Additionsbrechkraft, insbesondere eine diffraktive Additionsbrechkraft, $n_1$ den Brechungsindex des Kammerwassers, in dem die als Intraokularlinse ausgebildete Augenlinse sich befindet, $n_2$ den Brechungsindex des Materials der Intraokularlinse, insbesondere des Materials des optischen Teils. Darüber hinaus bedeutet ct die Mittendicke der Augenlinse entlang der Achse A, die hier beispielhaft 1 mm beträgt, $\lambda$ die zugrunde gelegte Designwellenlänge, die hier beispielhaft 546 nm beträgt, $\Delta\lambda$ einen

Phasenhub zwischen radial benachbarten Zonen, k die konische Konstante der Hüllasphäre, beziehungsweise "frac" den Flächenanteil einer Hauptunterzone an der gesamten Fläche der betrachteten Zone, rm den maximalen Radius des optischen Teils in Millimeter, $s_{max}$ die Zonenanzahl der optischen Zonen auf diesem Normradius.

Darüber hinaus bedeutet "cyl" beziehungsweise $F_{cyl}$ die Zylinderbrechkraft und somit den Brechkraftanteil an der Gesamtbrechkraft $F_{Ges}$ der Augenlinse, welcher durch einen torisch brechenden Oberflächenbereich beziehungsweise eine Oberflächenform beigetragen ist. "sph" beziehungsweise $F_{Sphäre}$ bezeichnet die sphärische Brechkraft, die anteilig zur Gesamtbrechkraft der Augenlinse beiträgt. Darüber hinaus bezeichnet "edof" beziehungsweise $F_{EDof}$ den Brechkraftanteil, der durch eine Helixwindung zur Gesamtbrechkraft der Augenlinse beigetragen wird. Darüber hinaus bezeichnen HOA1 und HOA2 beziehungsweise $F_{HOA1}$ und $F_{HOA2}$ Brechkraftanteile zur Gesamtbrechkraft der Augenlinse, die zur Korrektur von Aberrationen höherer Ordnung beitragen. Die Brechkräfte sind in Dioptrie angegeben.

Bei diesem ersten Ausführungsbeispiel wird somit eine multifokale Augenlinse erläutert, die neben ringförmig ausgebildeten Zonen, von denen die Zonen jeweils eine Hauptunterzone und eine Phasenunterzone aufweisen, zusätzlich eine Helixwindung aufweist, die einmal um die optische Hauptachse A umläuft. Bei der Ausgestaltung gemäß Fig. 2a bis 2h ist darüber hinaus der Phasenhub Φ konstant.

In Fig. 2b ist in einem Diagramm die Gesamtbrechkraft $F_{Ges}$ der Augenlinse 1 in Abhängigkeit vom Azimut eines Umlaufs dargestellt. Wie zu erkennen ist, steigt die Gesamtbrechkraft kontinuierlich an und ist darüber hinaus stufenlos.

In Fig. 2c ist demgegenüber ein Diagramm gezeigt, bei dem die azimutale Brechkraft $F_{EDof}$ in Abhängigkeit vom Azimut dargestellt ist. Durch diesen Brechkraftanteil wird die Schärfentiefe spezifisch eingestellt und erhöht. Wie aus dem Diagramm gemäß Fig. 2c zu entnehmen ist, ist auch hier ein stetig ansteigender Verlauf gegeben, der stufenlos ist, und in einem sprungartigen Übergang zwischen einem Windungsanfang und einem Windungsende der Helixwindung ist dann eine Brechkraftdifferenz von 1 Dioptrien erzeugt.

Durch den in Fig. 2c gezeigten Verlauf dieser azimutalen Brechkraftverteilung wird auch eine unterschiedliche Gewichtung in den einzigen Azimutwinkeln erreicht. Die zwei beim Windungsanfang und beim Windungsende vorliegenden Brechkräfte werden stärker gewichtet, da sie über einen größeren Winkelbereich anliegen beziehungsweise eine geringere Variation auftritt, als in einem dazwischen liegenden Winkelintervall.

In Fig. 2d ist ein Diagramm gezeigt, bei welchem die sagittale Höhe S und somit die Höhe in Richtung der optischen Achse A in Abhängigkeit vom Radius des optischen Teils 2 gezeigt ist. Es ist hier eine Schnittdarstellung der Seite 4 des optischen Teils 2 gezeigt, wobei nur eine Hälfte der gesamten geschnittenen Kontur gezeigt ist.

Wie zu erkennen ist, ist bei diesem Ausführungsbeispiel eine Mehrzahl von zumindest teilweise um die Achse A umlaufenden ringförmigen Zonen ausgebildet, von denen lediglich, der Übersichtlichkeit dienend, die von der Achse A nach außen betrachtet innenliegenden Zonen 6, 7, 8, 9, die radial direkt aneinander anschließen, mit einem Bezugszeichen versehen sind. Die Zone 6 weist eine Hauptunterzone 6a und eine Phasenunterzone 6b auf. Eine radial dann folgende Zone 7 weist ebenfalls eine Hauptunterzone 7a und eine Phasenunterzone 7b auf. Entsprechendes gilt für die Zonen 8 und 9, die dann ebenfalls Hauptunterzonen 8a und 9a sowie Phasenunterzonen 8b und 9b aufweisen.

Durch diese Ausgestaltung wird eine refraktiv-diffraktive Oberfläche geschaffen.

Beispielhaft ist auch noch eine Einhüllende 10 dargestellt, die die Spitzen der Phasenunterzonen 6b bis 9b sowie gegebenenfalls aller weiteren vorhandenen Phasenunterzonen verbindet.

Im Ausführungsbeispiel ist vorgesehen, dass der Flächenanteil der Hauptunterzonen 6a bis 9a bei den Zonen 6 bis 9 85 % der Gesamtfläche der jeweiligen Zonen 6 bis 9 beträgt.

Ein derartiger Flächenanteil kann auch noch größer sein, und es kann auch eine Ausgestaltung vorgesehen sein, bei der der Flächenanteil einer Phasenunterzone gleich 0 ist. Dadurch ist dann eine Kinoformlinse realisiert.

In Fig. 2d ist darüber hinaus auch ein Phasenhub Φ eingezeichnet, wie er zwischen zwei radial benachbarten und aneinander angrenzenden Zonen 6 und 7 bemessen ist. Entsprechend wird auch ein Phasenhub zwischen jeweils zwei anderen aneinander angrenzenden Zonen bestimmt. Vorzugsweise beträgt hier der Phasenhub Φ einen Wert, der kleiner der Designwellenlänge λ ist, insbesondere zwischen 0,3 λ und 0,6 λ beträgt.

Vorzugsweise ist bei dem Ausführungsbeispiel gemäß Fig. 2a bis 2h auch vorgesehen, dass eine relative Intensitätsverteilung eines Fernfokus und eines Nahfokus 65 % zu 35 % beträgt. Die als Intraokularlinse ausgebildete Augenlinse 1 ist bezüglich ihrer einhüllenden Topographie und somit der Einhüllenden 10 insbesondere auch spiegelsymmetrisch ausgelegt und aberrationsneutral asphärisiert.

Durch die in Fig. 2b dargestellte azimutal variierende Gesamtbrechkraft kann der Schärfentiefenwert erhöht werden, insbesondere an zumindest einem der genannten Foki der Augenlinse 1 erhöht werden.

Insbesondere ist vorgesehen, dass bei der Darstellung in Fig. 2c der azimutale Verlauf der durch die Helixwindung beitragenden Brechkraft $F_{EDof}$ beispielhaft eine Kosinusfunktion der Frequenz 0,5 ist. Es sind jedoch vielfältige andere Ausgestaltungen für den Kurvenverlauf der Brechkraft $F_{EDof}$ möglich.

In Fig. 2i ist ein Diagramm gezeigt, bei dem der Scheitelkrümmungsradius $r_s$ der Augenlinse in Abhängigkeit vom Azimut dargestellt ist. Ein Scheitel 11 des optischen Teils 2 ist in Fig. 1a und Fig. 1b beispielhaft dargestellt.

In dem Diagramm gemäß Fig. 2f ist die sagittale Höhe S in Abhängigkeit vom Radius r für das Ausführungsbeispiel gezeigt. Es ist an dieser Stelle zu erwähnen, dass die Darstellung in Fig. 2d lediglich die Strukturierung der Oberfläche darstellen soll und dahingehend die Ausgestaltung der Zonen 6 bis 9 sowie die Orientierungen der Hauptunterzonen 6a bis 9a und der Phasenunterzonen 6b bis 9b verdeutlichen soll. Die für das Ausführungsbeispiel wertmäßig genaue Darstellung ist in dem Diagramm gemäß Fig. 2f angegeben.

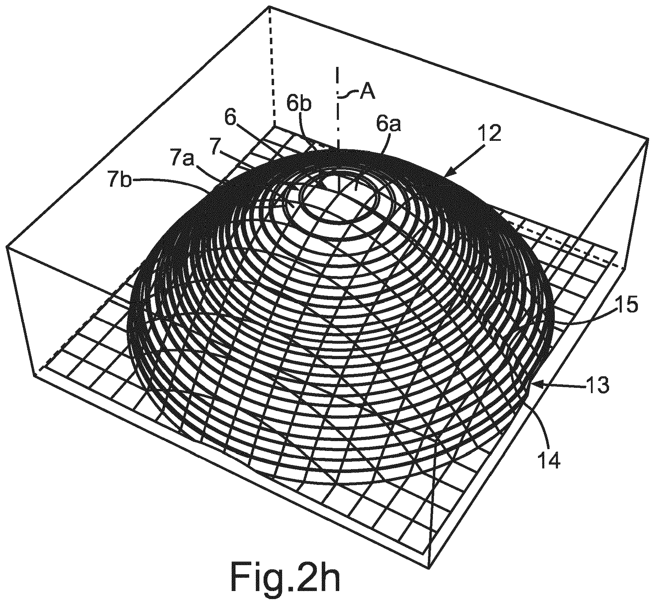

In Fig. 2h ist in einer schematischen Darstellung eine dreidimensionale Oberflächentopographie der Seite 4 gezeigt. Es ist hier zu erkennen, dass die ringförmigen Zonen 6 bis 9 zu erkennen sind.

Darüber hinaus ist auch die einmal um die optische Hauptachse A umlaufende Helixwindung 12 zu erkennen. Diese Helixwindung 12 weist einen Windungsanfang 14 und ein Windungsende 15 auf. Zwischen dem Windungsanfang 14 und dem Windungsende 15 ist ein sprungartiger Übergang 13 gestaltet. Dieser weist einen möglichst geringen azimutalen Winkelbereich auf, so dass er diesbezüglich möglichst klein und minimiert ausgebildet ist.

Der Windungsanfang 14 stellt zugleich auch einen Begrenzungsrand des Übergangs 13 dar, wobei Gleiches auch für das Windungsende 15 gilt. Im Ausführungsbeispiel stellt der Windungsanfang 14 einen radialen Verlauf dar, der flacher ist, als der radiale Verlauf des Windungsendes 15. Die sagittale Höhe S kann gemäß nachstehender Formel bestimmt werden:

$$s(r, r_0, Q) = \frac{r^2}{r_0\left(1 + \sqrt{1 - (1+Q)\frac{r^2}{r_0^2}}\right)} \qquad (3)$$

$r$ = radiale Position auf Linsenoberfläche
$r_0$ = zentraler Krümmungsradius ($r_0 \rightarrow r_{target}$ = Krümmungsradius der Zielfunktion (einhüllende Oberfläche))
Q bzw. k = konische Konstante

Diese Gleichung für die sagittale Höhe beschreibt den radialen Verlauf einer konischen Asphäre. Diese kann durch Addition von Polynomkoeffizienten verallgemeinert oder durch andere Beschreibungsarten, beispielsweise durch Zernike-Reihen, Polynome, Splines oder Bezierkurven ersetzt werden. Durch die Variation der Asphärizität, beispielsweise durch Variation der konischen Konstante Q bzw. k, kann der azimutabhängige radiale Brechkraftverlauf beeinflusst werden. Dadurch können auch Abbildungsfehler durch sphärische Aberration kompensiert werden. Die Berechnung der Sagittalhöhe S in jedem Azimut führt direkt zur einhüllenden dreidimensionalen Topographie der Linsenoberfläche und dient somit als Zielfunktion für die Berechnung der refraktiven Oberfläche. Diese einhüllende Zielfunktion ist durch die Einhüllende 10 in Fig. 2d verdeutlicht.

Durch die Helixwindung 12 wird ausgehend von dem Windungsanfang 14 und einem damit verknüpften Brechkraftwert ein kontinuierlicher Anstieg des Brechkraftwerts bis zum Windungsende 15 erreicht, wobei dies durch den beispielhaften Kurvenverlauf in Fig. 2c charakterisiert ist.

Wie bereits erwähnt, weist jede der konzentrischen und rotationssymmetrischen Hauptzonen 6a bis 9a in radialer Richtung einen Phasenhub Φ von vorzugsweise 0,46 λ zu seiner beidseitig benachbarten Hauptzone auf. Dies führt dann insbesondere zu der bereits erwähnten Intensitätsverteilung von 65 % zu 35 % zwischen dem Fernfokus und dem Nahfokus.

In Fig. 2g ist noch ein Diagramm gezeigt, in dem eine Fokusverschiebung und somit die Längenausdehnung beziehungsweise die Verbreiterung dargestellt ist, wodurch sich die Schärfentiefe in Vergrößerung des Fernfokus und des Nahfokus auch im Vergleich zu Ausgestaltungen ohne eine derartige Helixwindung erkennen lässt.

Aus Fig. 2g ist zu erkennen, dass bei einer diffraktiven Additionsbrechkraft von 3,75 Dioptrien und einer Pupillengröße von 3 mm, die dem maximalen Radius des optischen Teils 2 entspricht, ein Schärfentiefenbereich von im Wesentlichen 1,8 Dioptrien jeweils für den Nahfokus und den Fernfokus auftritt.

Gegenüber einer Linse aus dem Stand der Technik entspricht das einem wesentlichen Zugewinn an Schärfentiefe von circa 85 % bei einer Pupille von 3 mm. Dieser deutliche Zugewinn an Schärfentiefe reduziert die Empfindlichkeit der optischen Abbildungseigenschaften gegenüber Defokussierungsfehlern.

In Fig. 3a ist wiederum eine Parameterwertangabe von einem Parametersatz für ein weiteres Ausführungsbeispiel einer erfindungsgemäßen Augenlinse dargestellt.

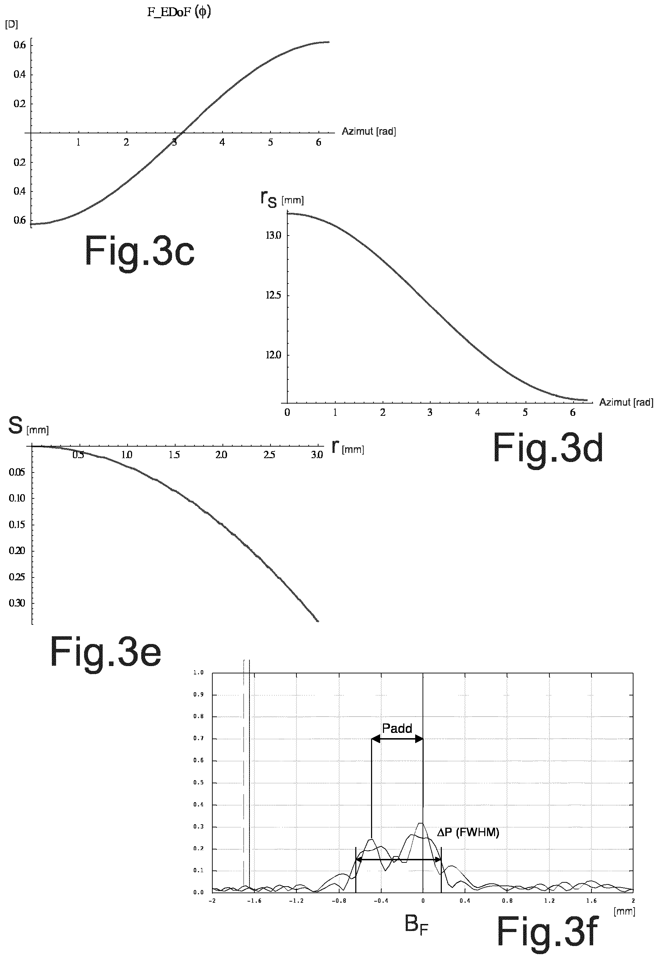

Bezüglich der Diagramme in Fig. 3b, 3c, 3d und 3e sind wiederum spezifische Parameter des Parametersatzes gemäß Fig. 3a in Abhängigkeit des Azimuts dargestellt.

In Fig. 3f ist wiederum die Breite $B_F$ durch die Angabe der Halbwertsbreite gekennzeichnet, so dass im Vergleich zum ebenfalls dargestellten Breitenwert im Stand der Technik die Verbreiterung durch das Ausführungsbeispiel der erfindungsgemäßen Augenlinse erkennbar ist.

Bei dem Ausführungsbeispiel gemäß Fig. 3a bis 3g ist somit ein Ausführungsbeispiel gezeigt, wie dies analog zu den Ausführungsbeispielen in Fig. 2a bis 2h neben den ringförmigen Zonen eine Helixwindung aufweist und bei welchen der Phasenhub zwischen zwei radial angrenzenden Zonen in azimutaler Richtung konstant ist. Dies gilt insbesondere dann auch für alle Zonen der Augenlinse 1.

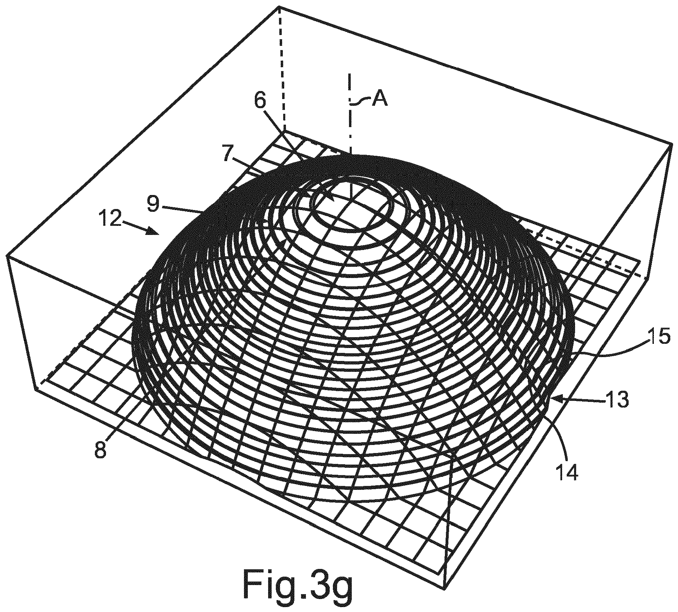

In Fig. 3g ist eine schematische dreidimensionale Oberflächentopographie der Seite 4 dieses Beispiels gezeigt.

In Fig. 4a bis 4e ist ein weiteres Ausführungsbeispiel mit konkreten Parameterwerten und Verläufen von Parametern in Abhängigkeit vom Azimut beziehungsweise vom Radius dargestellt. Bei diesem Ausführungsbeispiel umfasst die Augenlinse ringförmige Zonen und der Phasenhub Φ zwischen zwei radial benachbarten Zonen ist in azimutaler Richtung konstant. Dies ist auch bei den bisherigen Ausführungsbeispielen entsprechend vorliegend. Darüber hinaus ist beim Ausführungsbeispiel gemäß Fig. 4a bis 4e zusätzlich eine torisch brechende Oberflächenform 16 vorhanden. Bei diesem Ausführungsbeispiel gemäß Fig. 4a bis 4e ist jedoch keine Helixwindung ausgebildet. Eine derartige kann jedoch auch noch vorgesehen sein und beispielsweise mit dem torisch brechenden Oberflächenprofil beziehungsweise der Oberflächenform 16 und den ringförmigen Zonen 6 bis 9 auf einer Seite, beispielsweise der Seite 4, überlagert sein.

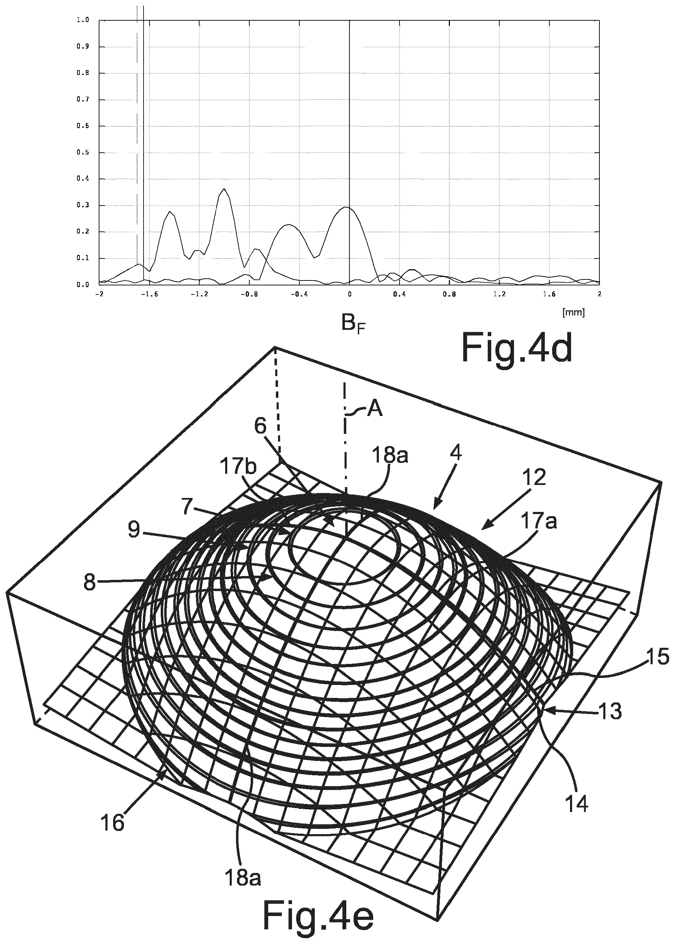

Wie in Fig. 4e beispielhaft dargestellt, weist diese torisch brechende Oberflächenform 16 senkrecht aufeinander stehende Hauptmeridiane 17a, 17b und 18a, 18b auf. Durch die Überlagerung der Helixwindung 12 weisen die Hauptmeridianabschnitte 17a und 17b unterschiedliche Radien, insbesondere Scheitelkrümmungsradien, auf. Entsprechendes gilt für die Hauptmeridianabschnitte 18a und 18b.

In der gezeigten Ausführung zeigen die Hauptmeridiane 15a und 15b die flacheren Abschnitte, wohingegen die Hauptmeridiane 16a und 16b die steileren darstellen.

In Fig. 4a bis 4e ist ein weiteres Ausführungsbeispiel einer Augenlinse 1, die als Intraokularlinse ausgebildet ist, gezeigt. Bei dieser Ausführung ist vorgesehen, dass neben den ringförmigen Zonen eine Helixwindung 12 und ein torisch brechendes Oberflächenprofil beziehungsweise eine torisch brechende Oberflächenform 16 vorhanden ist. Die Hauptmeridiane stehen hier senkrecht aufeinander und die Meridianabschnitte 17a und 17b weisen den flacheren Radius gegenüber den Meridianabschnitten 18a und 18b auf.

Im Übrigen sind dann durch die Diagramme gemäß Fig. 4b, 4c, 4d wiederum der Scheitelradius $r_s$, die Sagittalhöhe S in Abhängigkeit vom Azimut beziehungsweise vom Radius dargestellt, und die Fokusbreite $B_F$ ist gezeigt.

In Fig. 5a bis 5f ist ein weiteres Ausführungsbeispiel einer erfindungsgemäßen Augenlinse 1 erläutert. Bei dieser Ausführung ist neben ringförmigen Zonen eine torisch brechende Oberflächenform 16 ausgebildet. Bei diesem Ausführungsbeispiel ist ebenfalls der Phasenhub zwischen zwei benachbarten Zonen 6 bis 9 in azimutaler Richtung jeweils konstant. Bei diesem Ausführungsbeispiel weist die Augenlinse keine Helixwindung auf. Es kann jedoch auch hier eine Ausführung vorgesehen sein, die aufbauend auf den zugrunde gelegten Parametern und Parameterwerten auch zusätzlich eine Helixwindung aufweist und/oder ein Phasenhub Φ in azimutaler Richtung variiert.

In Fig. 5g ist wieder eine schematische dreidimensionale Darstellung der Oberflächengeometrie einer Seite 4 des optischen Teils 2 dargestellt, wobei auch hier alle Profilanteile auf dieser Seite 4 ausgebildet sind.

Bei dieser Ausgestaltung sind die Hauptmeridiane, der flache Hauptmeridian 17 und der steile Hauptmeridian 18 jeweils links- und rechtsseitig von der optischen Hauptachse A gleich gestaltet, so dass keine Abschnitte mit unterschiedlichen Krümmungsradien, insbesondere Scheitelkrümmungsradien, wie dies in Fig. 4e gezeigt ist, vorhanden sind.

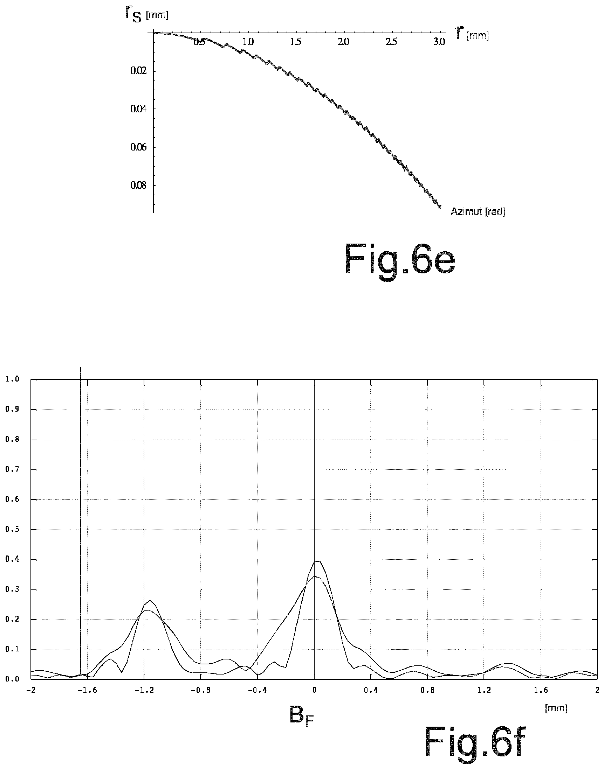

In Fig. 6a bis 6g ist ein weiteres Ausführungsbeispiel einer Augenlinse gezeigt. Bei dieser Ausführung weist die Augenlinse 1 einen Phasenhub Φ auf, wie er im Verlauf gemäß Fig. 6c gezeigt ist. Die grundlegenden Parameterwerte gemäß der Auflistung in Fig. 6a sind mit einigen Parametern dann im Graphen gemäß den Diagrammen in Fig. 6b, 6c, 6d, 6e und 6f dargestellt.

Der Phasenhub variiert bei dieser Ausgestaltung beispielhaft gemäß dem Kurvenverlauf in Fig. 6c und steigt somit kontinuierlich an. Ausgang ist hier ein spezifischer Anfang in azimutaler Richtung.

Bei dem Ausführungsbeispiel in Fig. 6a bis 6g sind lediglich ringförmige Zonen 6 bis 9 ausgebildet, ansonsten ist jedoch keine Helixwindung oder eine torisch brechende Oberflächenform ergänzend ausgebildet beziehungsweise überlagert. Es kann jedoch auch eine Ausführung vorgesehen sein, bei der eine Helixwindung und/oder eine torisch brechende Oberflächenform ebenfalls zusätzlich vorhanden sind.

In Fig. 6f ist wiederum die Fokusverbreiterung der Foki der insbesondere bifokalen Linse gezeigt.

Auch hier soll erwähnt werden, dass bei allen Ausführungen auch eine trifokale, quadrafokale oder darüber hinausgehend mehrfokalige Augenlinse ausgebildet sein kann.

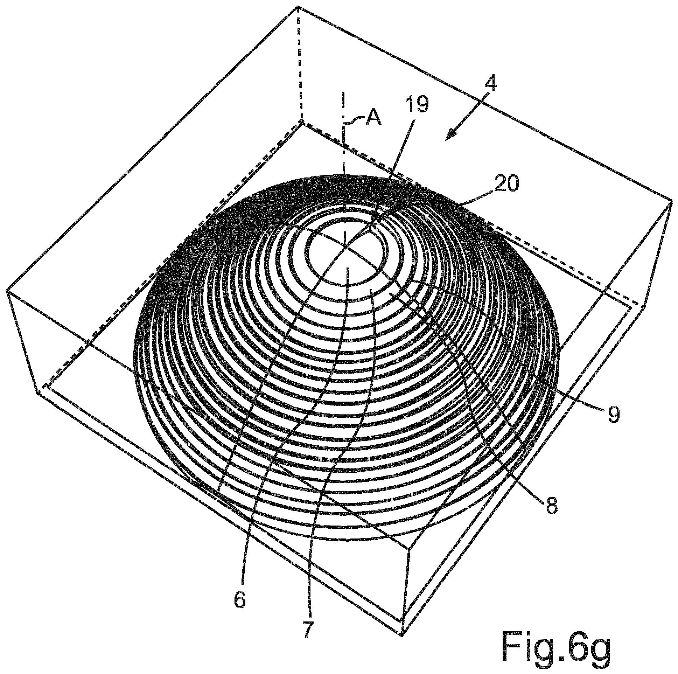

Der Verlauf der sagittalen Höhe S in Abhängigkeit vom Radius r ist im Diagramm gemäß Fig. 6e dargestellt. Darüber hinaus ist in Fig. 6g eine schematische Darstellung einer dreidimensionalen Oberflächentopographie des Ausführungsbeispiels gezeigt. An einem azimutalen Anfang 19 ist zwischen den Zonen 6 bis 9, die beispielhaft für alle weiteren Zonen lediglich mit einem Bezugszeichen versehen sind, der minimale Phasenhub von 0 ausgebildet, der sich dann im Ausführungsbeispiel entgegen dem Uhrzeigersinn um die Achse A laufend kontinuierlich erhöht und dann an einem Ende 20 das Maximum erreicht. Dieser Phasenhubverlauf ist sowohl in einer Hauptunterzone als auch in einer Phasenunterzone der Zonen 6 bis 9 gleich ausgebildet. Dies gilt insbesondere für die ersten inneren Zonen der Augenlinse 1, insbesondere für alle Zonen der Augenlinse 1.

Ein weiteres Ausführungsbeispiel ist in den Fig. 7a bis 7i gezeigt, wobei hier im Unterschied zur Ausgestaltung gemäß Fig. 6a bis 6g die Variation des Phasenhubs Φ unterschiedlich ist. Darüber hinaus sind auch die Parameterwerte

der Grundbrechkraft $P_0$, der sphärischen Brechkraft $F_{Sphäre}$ und den Normradius rmmax und rmmin (beide in Millimeter angegeben) gegenüber dem Ausführungsbeispiel in Fig. 6a bis 6g verändert. In Fig. 6a und 7a bezeichnen "phasemin" und "phasemax" den minimalen Phasenhub $\Phi$ und den maximalen Phasenhub $\Phi$ bei einem Umlauf um die Achse A.

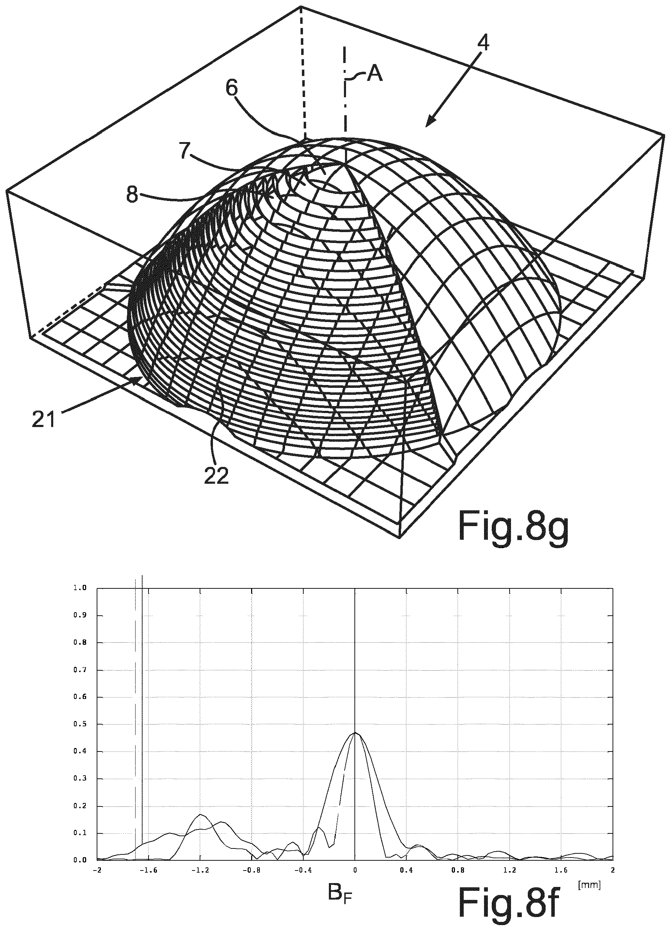

In dem Ausführungsbeispiel gemäß Fig. 8a bis 8f ist wiederum ein Beispiel gezeigt, bei dem der Phasenhub in azimutaler Richtung variiert, ansonsten jedoch keine Helixwindung und keine torisch brechende Oberflächenform ausgebildet ist. Bei dieser Ausgestaltung ist der Phasenhub diskret in einem spezifischen Azimutintervall gestaltet, wie dies in Fig. 8c dargestellt ist. Sowohl die azimutale Lage als auch die Höhe des Phasenhubs ist lediglich beispielhaft zu verstehen. Auch ist die Breite des Azimutintervalls, über welche dieser maximale Phasenhub auftritt, lediglich beispielhaft. Durch ein derartiges Ausführungsbeispiel, bei dem in Fig. 8g die beispielhafte dreidimensionale Oberflächentopographie dargestellt ist, kann auch die Ausgestaltung von kuchenstückartigen Oberflächensektoren spezifischer Strukturierung erreicht werden. Bei dieser Ausführung ist ein kuchenstückartiger Oberflächensektor 21 realisiert, bei dem sich die Zonen 6, 7, 8 und 9 über ein Winkelintervall von etwa 120° erstrecken. Wie darüber hinaus in Fig. 8g zu erkennen ist, ist die restliche Oberfläche der Seite 4 unstrukturiert und somit glatt ausgebildet.

In Fig. 8e ist eine Schnittdarstellung der Topographie in Fig. 8g gezeigt, wobei dabei die sagittale Höhe in Abhängigkeit vom Radius gezeigt ist und dabei entlang des Radius 22 verläuft. Auch hier sind die Zonen mit jeweils einer Hauptunterzone und einer Phasenunterzone ausgebildet, wobei in Fig. 8a lediglich die innerste Zone 6 mit der Hauptunterzone 6a und der Phasenunterzone 6b dargestellt ist.

In den Fig. 8b bis 8g sind wieder spezifische Parameter mit ihren charakteristischen Verläufen über den Azimut dargestellt.

In Fig. 8f ist wiederum die Fokusverbreiterung $B_F$ des Ausführungsbeispiels gegenüber einer Ausgestaltung ohne einer derartigen Variation des Phasenhubs gezeigt. Auch hier sind die beiden Foki in ihrer Breite vergrößert, so dass auch in den jeweiligen Foki eine erhöhte Schärfentiefe vorliegt.

Durch die Verhältnisbildung zwischen dem Winkelintervall, in dem der Phasenhub 0 ist, und in dem der Phasenhub > 0 ist, kann auch die Intensitätsverteilung zwischen dem Fernfokus und dem Nahfokus eingestellt werden. Der Nahfokus ist insbesondere durch den Bereich charakterisiert, bei dem der Phasenhub $\Phi$ > 0 ist und somit durch den kuchenstückförmigen Oberflächensektor 22 charakterisiert.

Im Ausführungsbeispiel gemäß Fig. 9a bis 9h ist eine Augenlinse 1 beispielhaft gezeigt, bei der neben einer Variation des Phasenhubs $\Phi$ zu den ringförmigen Zonen auch zusätzlich eine torisch brechende Oberflächenform 16 ausgebildet ist.

Wie durch die beispielhaften Parameterwerte und die beispielhaften Verläufe der Parameter in Abhängigkeit vom Azimut in Fig. 9a bis 9f gezeigt ist, wird hier eine ganz spezifische Oberflächentopographie, wie sie in Fig. 9h dreidimensional gezeigt ist, erreicht. Die Zylinderbrechkraft, wie sie durch die torisch brechende Oberflächenform 16 gemäß dem Verlauf in Fig. 9c zur Gesamtbrechkraft beigetragen wird, und/oder durch den gleichlaufenden Kurvenverlauf des Phasenhubs, wie er in Fig. 9d dargestellt ist, wird der spezifische Brechkraftverlauf der Gesamtbrechkraft in Fig. 9b erreicht. Insbesondere ist bei diesem Ausführungsbeispiel der Phasenhub im Bereich des Hauptmeridians 17, der den flacheren Hauptmeridian darstellt, gleich 0. An dieser Stelle ist somit die Topographie glatt und strukturiert sich bezüglich der Zonen dann erst im und gegen den Uhrzeigersinn dazu entsprechend.

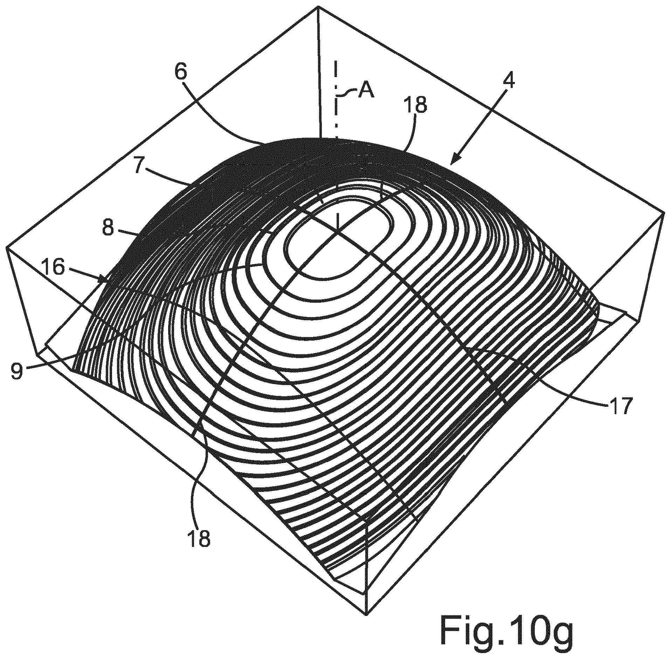

In Fig. 10a bis 10g ist ein weiteres Ausführungsbeispiel gezeigt, bei dem in Analogie zur Ausgestaltung in Fig. 9a bis 9h ebenfalls neben den Ausgestaltungen einer ringförmigen Zone mit jeweils einer Hauptunterzone und einer Phasenunterzone eine azimutale Variation des Phasenhubs erfolgt und darüber hinaus eine torisch brechende Oberflächenform 16 überlagert ist, wie dies in Fig. 10g dargestellt ist.

Eine Helixwindung ist hier ebenfalls nicht ausgebildet, kann jedoch hier als auch bei dem Ausführungsbeispiel gemäß Fig. 9a bis 9h ebenfalls überlagert vorgesehen sein.

Bei dieser Ausgestaltung ist in azimutaler Richtung wieder ein relativ kleiner Phasenhub $\Phi$ realisiert, wie er auch in dem Diagramm gemäß Fig. 10g dargestellt ist. Der Verlauf der Gesamtbrechkraft in Abhängigkeit vom Azimut ist in Fig. 10b dargestellt.

In Fig. 10c ist der azimutale Verlauf des Normierungsradius für die diffraktive Struktur gezeigt, der beispielhaft einen Verlauf mit Tälern und Bergen aufweist.

Der minimale Normradius rmmin beträgt hier 3 mm und der maximale Normradius rmmax beträgt hier 4,5 mm.

Dadurch ist auch die torisch brechende Oberflächenform 16 charakterisiert.

Durch den Hauptmeridian 17 ist der flachere Hauptmeridian gezeigt, wobei der dazu senkrecht stehende Hauptmeridian 18 den steileren darstellt.

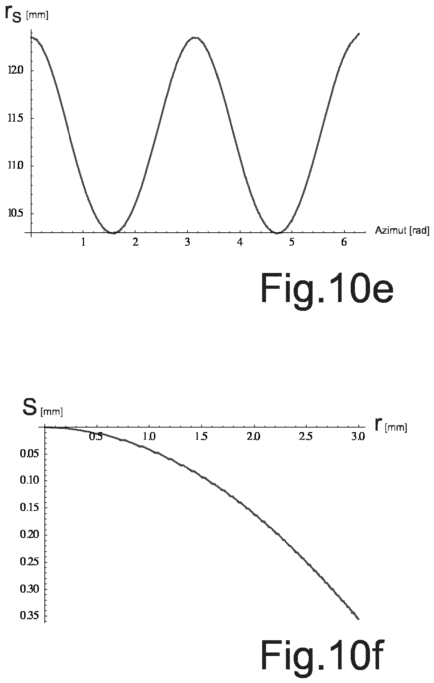

In den weiteren Diagrammen gemäß Fig. 10e und 10f ist der Scheitelkrümmungsradius für die sagittale Höhe in Abhängigkeit vom Azimut beziehungsweise vom Radius dargestellt.

Bei dem Ausführungsbeispiel gemäß Fig. 11a bis 11h ist eine Augenlinse dargestellt, bei der der Phasenhub in azimutaler Richtung wiederum variiert. Darüber hinaus sind hier keine Helixwindung und auch keine torisch brechende Oberflächenform gestaltet. Allerdings ist hier vorgesehen, dass der Radius der ringförmigen Zonen in azimutaler Richtung zumindest einmal variiert.

In Fig. 11a ist dabei wieder ein beispielhafter Parameterwertesatz für ein Ausführungsbeispiel angegeben. In den Diagrammen gemäß Fig. 11a bis 11f sind wiederum spezifische Parameter in Abhängigkeit vom Azimut beziehungsweise vom Radius dargestellt. Insbesondere in Fig. 11d ist in dem Zusammenhang die azimutale Radiusvariation gezeigt, die hier symmetrisch einem Kurvenverlauf mit Tälern und Bergen gleicht. Dieser Verlauf ist im Ausführungsbeispiel für die zumindest inneren Zonen zugrunde zulegen, so dass diese in radialer Richtung jeweils an gleicher azimutaler Stellung die jeweiligen unterschiedlichen Radien aufweisen.

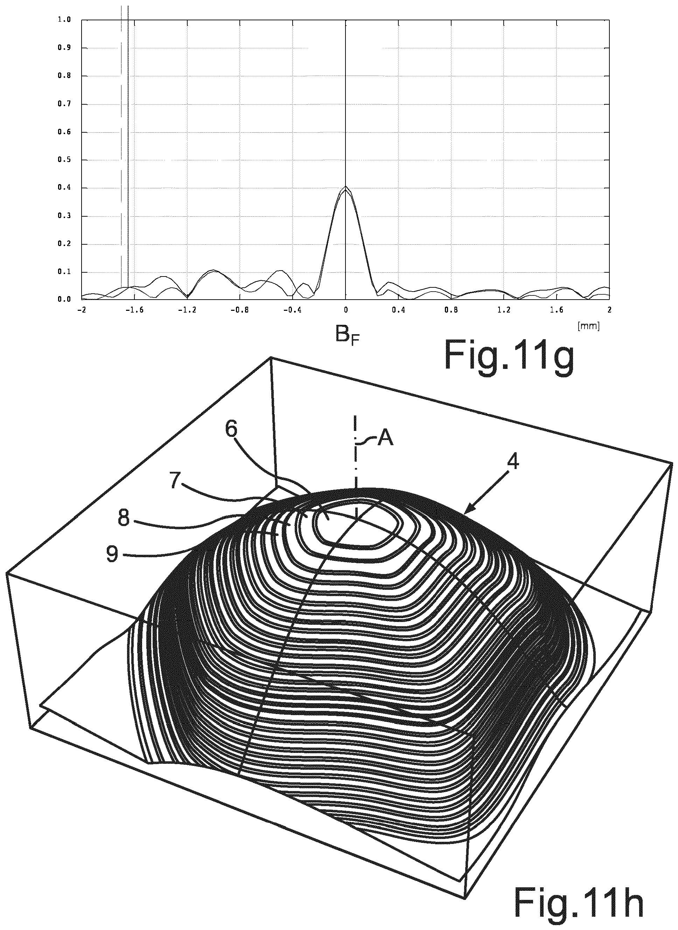

In Fig. 11g ist wiederum die Breite der Foki der Linse gezeigt.

In dem dreidimensionalen Bild in Fig. 11h ist die Oberflächentopographie des Ausführungsbeispiels gezeigt. Der Verlauf der einzelnen Zonen in azimutaler Richtung ist zu erkennen, wobei dies bei einer Betrachtung entlang der Achse A derart gestaltet ist, dass diese Zonen 6 bis 9 ähnlich einem Polygonring gestaltet sind. Bei dem gezeigten Ausführungsbeispiel sind keine Helixwindung und keine torisch brechende Oberflächenform ausgebildet beziehungsweise überlagert.

Es kann jedoch auch hier vorgesehen sein, dass auch eine torisch brechende Oberflächenform vorhanden ist, beispielsweise mit einem Brechkraftanteil zwischen 1 Dioptrien und 3 Dioptrien, insbesondere 2 Dioptrien.

Diese beispielhaften Werte können auch bei den anderen Ausführungen, die bisher ohne torische Oberflächenform erläutert wurden, ausgebildet sein, wenn eine torische Oberflächenform zusätzlich ausgebildet ist.

In Fig. 12a bis 12g ist ein weiteres Ausführungsbeispiel einer Augenlinse 1 gezeigt, bei welcher im Unterschied zur Ausführung in Fig. 11a der maximale Normradius rmmax größer ist. Darüber hinaus ist der Verlauf dieses Normierungsradius in Fig. 12d dargestellt, der keinen Verlauf mit mehreren Tälern und Bergen aufweist, sondern stetig ansteigend ausgebildet ist.

In den Fig. 12b, 12c, 12e und 12f sind wieder spezifische Kurvenläufe für spezifische Parameter in Abhängigkeit vom Azimut beziehungsweise vom Radius dargestellt.

In Fig. 12g ist beispielhaft und schematisch die dreidimensionale Oberflächentopographie zu diesem Ausführungsbeispiel gezeigt.

Bei dem Ausführungsbeispiel in Fig. 13a bis 13g ist im Unterschied zu den Ausführungen in Fig. 11a bis 11h und 12a bis 12g der maximale Normierungsradius rmmax wiederum vergrößert und der azimutale Kurvenverlauf gemäß der Darstellung in Fig. 13c ähnlich zum Verlauf in Fig. 12d. Die weiteren Diagramme zeigen auch hier dann wiederum Darstellungen spezifischer Parameter in Abhängigkeit vom Azimut beziehungsweise vom Radius, sowie die Darstellung der Fokusbreite.

Ein abschließendes Beispiel ist in den Fig. 14a bis 14h dargestellt. Bei dieser Ausführung ist wiederum eine azimutale Variation des Phasenhubs Φ vorgesehen. Neben den ringförmigen Zonen, die auch hier wiederum beispielsweise jeweils eine Hauptunterzone und eine Phasenunterzone aufweisen, ist keine Helixwindung und auch keine torisch brechende Oberflächenform als zusätzliche zur optischen Abbildungseigenschaft beitragende Struktur ausgebildet. Es kann jedoch auch hier vorgesehen sein, dass eine einmal um die optische Achse A umlaufende Helixwindung und/oder eine torisch brechende Oberflächenform ausgebildet beziehungsweise überlagert sind.

Bei diesem Ausführungsbeispiel ist gemäß der Darstellung in Fig. 14b auch wiederum ein konstanter Verlauf der Gesamtbrechkraft abhängig vom Azimut dargestellt.

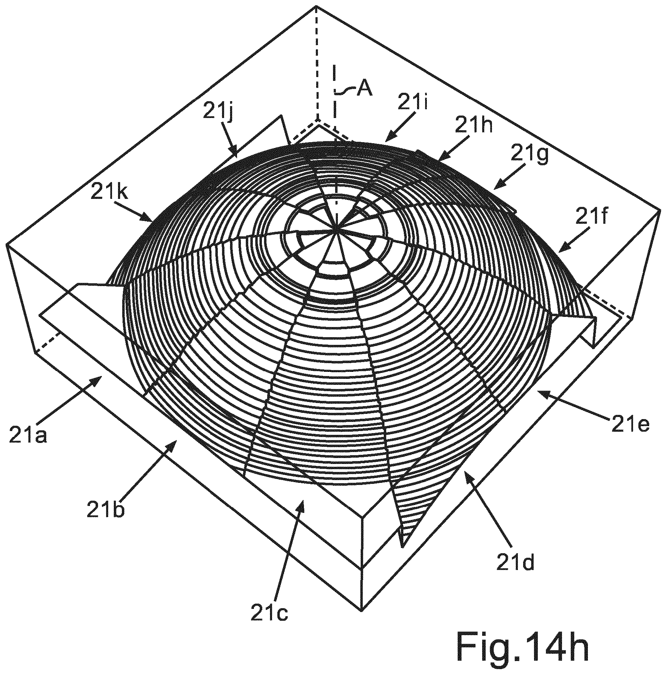

Der Verlauf des Normierungsradius, wie er in Fig. 14c gezeigt ist, ist hier ganz spezifisch, und zwar durch ein gestuftes diskretes Profil. Die mehrmalige Stufung führt dazu, dass gemäß der dreidimensionalen Darstellung der Oberflächentopographie in Fig. 14h mehrere kuchenstückartige Oberflächensektoren 21a, 21b, 21c, 21d, 21e, 21f, 21g, 21h, 21i, 21j und 21k gebildet sind. Gemäß der spezifischen Verlaufsvorgabe, wie sie in Fig. 14c gezeigt ist, erstrecken sich diese Oberflächensektoren 21a bis 21k jeweils über einen gleichen azimutalen Winkelbereich. Darüber hinaus sind insbesondere diese Oberflächensektoren 21a bis 21k in der Anzahl der jeweilig radial ausgebildeten Zonen und/oder der radialen Breite dieser Zonen und/oder den Flächengrößen dieser Zonen und/oder den Phasenhüben dieser Zonen unterschiedlich. Dies wird insbesondere im Hinblick auf den Phasenhub auch durch den Verlauf in Fig. 14d gezeigt.

Es können durch diese spezifische Ausgestaltung also äußerst individuelle Oberflächentopographien für Augenlinsen erzeugt werden, die sektorial individuell und insbesondere auch unabhängig von anderen Sektoren gestaltet werden können. Dadurch können ganz individuelle Abbildungseigenschaften erreicht werden.

Bei allen erläuterten Ausführungsbeispielen mit den konkreten Zahlenwerten kann auch vorgesehen sein, dass jeweils zumindest ein Parameterwert eines Parameters geändert wird und/oder zumindest ein Kurvenverlauf eines Parameters anderweitig gestaltet ist. Auch können bei den einzelnen konkret erläuterten Ausführungsbeispielen auch die Zonengestaltungen bezüglich der Anzahl der Hauptunterzonen und/oder der Anzahl der Phasenunterzonen und/oder bezüglich der Flächenanteile einer Hauptunterzone und/oder einer Phasenunterzone unterschiedlich sein. Ebenso kann vorgesehen sein, dass bei Ausführungen, die keine Helixwindung aufweisen, auch eine derartige Helixwindung ausgebildet ist. Ebenso kann bei Ausführungen, die keine torisch brechende Oberflächenform aufweisen, auch eine derartige noch ausgebildet sein. Ebenso kann bei Ausführungen, bei denen der Phasenhub konstant in azimutaler Richtung ist, eine Variation in azimutaler Richtung vorliegen. Es ist somit auch eine Vielzahl von weiteren Ausführungsbeispielen als offenbart anzusehen, die durch die oben genannten Veränderungen von Parametern und/oder Oberflächentopographien aus den erläuterten Ausführungsbeispielen hervorgehen.

Ganz allgemein ist auch zu erwähnen, dass bei allen Ausführungsbeispielen auch eine Aufteilung eines Teilprofils, welches zur Gesamtbrechkraft der Augenlinse beiträgt, auch auf beide Seiten 4 und 5 verteilt werden kann und/oder die jeweiligen Profile auf unterschiedlichen Seiten 4 und 5 ausgebildet sein können.

**Patentansprüche**

1. Multifokale Intraokularlinse (1) mit einem optischen Teil (2), der eine in Richtung einer optischen Hauptachse (A) der Intraokularlinse (1) betrachtet erste optische Seite (4) und eine gegenüberliegende zweite optische Seite (5) aufweist, und eine Mehrzahl von um die optische Hauptachse (A) zumindest teilweise umlaufende, auf zumindest einer Seite (4, 5) ausgebildete, ringförmige optische Zonen (6 bis 9) aufweist, und die Zonen (6 bis 9) jeweils zumindest eine Hauptunterzone (6a bis 9a) aufweisen, wobei zumindest einige Zonen (6 bis 9) zusätzlich zur Hauptunterzone (6a bis 9a) noch eine Phasenunterzone (6b bis 9b) aufweisen, wobei die Phasenunterzonen (6b bis 9b) Phasenunterzonen-Brechkräfte aufweisen und durch die Phasenunterzonen (6b bis 9b) Phasenverschiebungen zwischen Partialwellen aus verschiedenen ringförmigen Zonen (6 bis 9) erzeugt sind, wobei die Hauptunterzonen (6a bis 9a) zumindest teilweise um die optische Hauptachse (A) umlaufende, ringförmige Gebilde sind und die Phasenunterzonen (6b bis 9b) zumindest teilweise um die optische Hauptachse (A) umlaufende, ringförmige Gebilde sind, und eine Hauptunterzone (6a bis 9a) in radialer Richtung zur optischen Hauptachse (A) direkt an eine Phasenunterzone (6b bis 9b) einer ringförmigen Zone (6 bis 9) angrenzt, **dadurch gekennzeichnet, dass** die Intraokularlinse (1) zusätzlich zu den ringförmigen Zonen (6 bis 9) eine Helixwindung (12) als Oberflächenstruktur aufweist, wobei durch die Helixwindung (12) eine Brechkraft der Intraokularlinse (1) in Umlaufrichtung um die Hauptachse (A) wertmäßig variiert, und die Helixwindung (12) auf zumindest einer Seite (4, 5) ausgebildet ist, wobei eine durch die Hauptunterzone (6a bis 9a) zur Gesamtbrechkraft der Intraokularlinse (1) beitragende Hauptunterzonen-Brechkraft in azimutaler Richtung um die Hauptachse (A) zumindest einmal variiert und/oder eine durch die Phasenunterzone (6b bis 9b) zur Gesamtbrechkraft der Intraokularlinse (1) beitragende Phasenunterzonen-Brechkraft in azimutaler Richtung um die Hauptachse (A) zumindest einmal variiert, und ein Phasenhub ($\Phi$) zwischen zwei benachbarten Zonen (6 bis 9) im Intervall < $\lambda$, insbesondere im Intervall $0,3\lambda \leq \Phi(\alpha) \leq 0,6\lambda$, liegt, wobei der Phasenhub ($\Phi$) zumindest bei einer Zone (6 bis 9) in azimutaler Richtung um die Hauptachse (A) variiert ist, wobei der Phasenhub ($\Phi$) über ein erstes Teilazimutintervall gleich Null und über ein zweites Teilazimutintervall ungleich Null ist und abhängig von dem Verhältnis zwischen dem ersten und dem zweiten Teilazimutintervall ein Verhältnis zwischen einer Fernbrechkraft und einer Nahbrechkraft der Augenlinse (1) veränderbar ist.

2. Intraokularlinse (1) nach Anspruch 1, **dadurch gekennzeichnet, dass** die ringförmigen Zonen (6 bis 9) und die Helixwindung (12) auf einer gemeinsamen Seite (4, 5) ausgebildet und überlagert sind.

3. Intraokularlinse (1) nach Anspruch 1, **dadurch gekennzeichnet, dass** die ringförmigen Zonen (6 bis 9) auf einer Seite (4, 5) und die Helixwindung (12) auf der anderen Seite (4, 5) ausgebildet sind.

4. Intraokularlinse (1) nach einem der vorhergehenden Ansprüche, **dadurch gekennzeichnet, dass** der Phasenhub ($\Phi$) zumindest bei einer Zone (6 bis 9) in azimutaler Richtung um die Hauptachse (A) konstant ist.

5. Intraokularlinse (1) nach einem der vorhergehenden Ansprüche, **dadurch gekennzeichnet, dass** der Phasenhub ($\Phi$) stufenlos variiert ist.

6. Intraokularlinse (1) nach einem der vorhergehenden Ansprüche 1 bis 4, **dadurch gekennzeichnet, dass** der Phasenhub ($\Phi$) zumindest einmal durch eine diskrete Phasenhubstufe variiert ist.

7. Intraokularlinse (1) nach einem der vorhergehenden Ansprüche, **dadurch gekennzeichnet, dass** die Helixwindung (12) einmal um die Hauptachse (A) umlaufend ausgebildet ist und zwischen einem Windungsanfang (14) und einem Windungsende (15) ein sprungartiger Übergang (13) ausgebildet ist.

8. Intraokularlinse (1) nach einem der vorhergehenden Ansprüche, **dadurch gekennzeichnet, dass** die Intraokularlinse (1) zusätzlich zu den ringförmigen Zonen (6 bis 9) und der Helixwindung (12) eine torisch brechende Oberflächenform (16) aufweist, und die torisch brechende Oberflächenform (16) auf zumindest einer Seite (4, 5) ausgebildet ist.

9. Intraokularlinse (1) nach Anspruch 7 und 8, **dadurch gekennzeichnet, dass** der Übergang (13) in azimutaler Richtung gegenüber einem flachen Hauptmeridian (17, 17a, 17b) der torisch brechenden Oberflächenform (16) versetzt ist, insbesondere der sprungartige Übergang (13) in azimutaler Lage um die optische Hauptachse (A) in einem azimutalen Winkel zwischen 20° und 70°, insbesondere zwischen 40° und 50°, oder zwischen 200° und 250°, insbesondere zwischen 220° und 230°, zu einem flachen Hauptmeridian (17, 17a, 17b) der torisch brechenden Oberflächenform (16) ausgebildet ist, insbesondere der Versatz zwischen (1/4 π) +/- (1/8 π) oder (5/4 π) +/- (1/8 π) beträgt.

10. Intraokularlinse (1) nach einem der vorhergehenden Ansprüche, **dadurch gekennzeichnet, dass** sie n ≥ 2 Foki aufweist und die Helixwindung (12) so ausgebildet ist, dass maximal n-1 Foki durch die Helixwindung (12) in der Schärfentiefe vergrößert sind.

11. Intraokularlinse (1) nach einem der vorhergehenden Ansprüche, **dadurch gekennzeichnet, dass** eine Gesamtbrechkraft der Intraokularlinse (1) in azimutaler Richtung bei einem Umlauf um die Hauptachse (A) Zwischenminimumlos ansteigend variiert ist.

**Claims**

1. Multifocal intraocular lens (1) comprising an optical part (2), which has a first optical side (4) and an opposite second optical side (5) viewed in the direction of a main optical axis (A) of the intraocular lens (1), and a plurality of ring-shaped optical zones (6 to 9) which are formed on at least one side (4, 5) and which at least partly encircle the main optical axis (A), said zones (6 to 9) each having at least one main sub-zone (6a to 9a), wherein at least some zones (6 to 9) have a phase sub-zone (6b to 9b) in addition to the main sub-zone (6a to 9a), wherein the phase sub-zones (6b to 9b) have phase sub-zone refractive powers and phase shifts between partial waves from different ring-shaped zones (6 to 9) are produced by the phase sub-zones (6b to 9b), wherein the main sub-zones (6a to 9a) are ring-shaped forms which at least partly encircle the main optical axis (A) and the phase sub-zones (6b to 9b) are ring-shaped forms which at least partly encircle the main optical axis (A) and a main sub-zone (6a to 9a) directly adjoins a phase sub-zone (6b to 9b) of a ring-shaped zone (6 to 9) in the radial direction with respect to the main optical axis (A), **characterized in that** the intraocular lens (1) has a helix winding (12) as a surface structure in addition to the ring-shaped zones (6 to 9), wherein the refractive power of the intraocular lens (1) varies in terms of value in encircling direction around the main axis (A) by the helix winding (12) and the helix winding (12) is formed on at least one side (4, 5), wherein a main sub-zone refractive power contributing to the overall refractive power of the intraocular lens (1) by the main sub-zone (6a to 9a) varies at least once in azimuthal direction around the main axis (A) and/or a phase sub-zone refractive power contributing to the overall refractive power of the intraocular lens (1) by the phase sub-zone (6b to 9b) varies at least once in azimuthal direction around the main axis (A), and a phase deviation ($\Phi$) between two adjacent zones (6 to 9) is in the interval of < $\lambda$, in particular in the interval of $0.3\lambda \leq \Phi(\alpha) \leq 0.6\lambda$, wherein the phase deviation ($\Phi$) is varied at least in one zone (6 to 9) in azimuthal direction around the main axis (A), wherein the phase deviation ($\Phi$) is equal to zero over a first partial azimuthal interval and unequal to zero over a second partial azimuthal interval and a ratio between a distance refractive power and a near refractive power of the eye lens (1) is variable depending on the ratio between the first and the second partial azimuthal interval.

2. Intraocular lens (1) according to Claim 1, **characterized in that** the ring-shaped zones (6 to 9) and the helix winding (12) are formed and superimposed on a common side (4, 5).

3. Intraocular lens (1) according to Claim 1, **characterized in that** the ring-shaped zones (6 to 9) are formed on one side (4, 5) and the helix winding (12) is formed on the other side (4, 5).

4. Intraocular lens (1) according to any one of the preceding claims, **characterized in that** the phase deviation ($\Phi$) is constant at least in one zone (6 to 9) in azimuthal direction around the main axis (A).

5. Intraocular lens (1) according to any one of the preceding claims, **characterized in that** the phase deviation ($\Phi$) is continuously varied.

6. Intraocular lens (1) according to any one of the preceding Claims 1 to 4, **characterized in that** the phase deviation ($\Phi$) is varied at least once by a discrete phase deviation step.

7. Intraocular lens (1) according to any one of the preceding claims, **characterized in that** the helix winding (12) is formed once encircling the main axis (A) and a jump-like transition (13) is formed between a winding start (14) and a winding end (15).

8. Intraocular lens (1) according to any one of the preceding claims, **characterized in that** the intraocular lens (1) has a toric refractive surface shape (16) in addition to the ring-shaped zones (6 to 9) and the helix winding (12), and the toric refractive surface shape (16) is formed on at least one side (4, 5).

9. Intraocular lens (1) according to Claims 7 and 8, **characterized in that** the transition (13) is offset in azimuthal direction with respect to a flat main meridian (17, 17a, 17b) of the toric refractive surface shape (16), in particular the jump-like transition (13) is formed at an azimuthal angle between 20° and 70°, in particular between 40° and 50°, or between 200° and 250°, in particular between 220° and 230°, to a flat main meridian (17, 17a, 17b) of the toric refractive surface shape (16) in azimuthal position around the main optical axis (A), in particular the offset is between (1/4 $\pi$) +/-(1/8 $\pi$) or (5/4 $\pi$) +/- (1/8 $\pi$).

10. Intraocular lens (1) according to any one of the preceding claims, **characterized in that** it has n ≥ 2 foci and the helix winding (12) is formed such that at most n-1 foci are increased in the depth of focus by the helix winding (12).

11. Intraocular lens (1) according to any one of the preceding claims, **characterized in that** an overall refractive power of the intraocular lens (1) is varied in increasing manner in azimuthal direction without intermediate minimum in a tour around the main axis (A).

**Revendications**

1. Lentille intraoculaire multifocale (1) comprenant une partie optique (2) qui comporte une première face optique (4), vue dans la direction d'un axe optique principal (A) de la lentille intraoculaire (1) et une deuxième face optique opposée (5), et une pluralité de zones optiques annulaires (6 à 9) qui s'étendent au moins partiellement autour de l'axe optique principal (A) et qui sont formées sur au moins une face (4, 5), et les zones (6 à 9) comportant chacune au moins une sous-zone principale (6a à 9a), au moins certaines zones (6 à 9) comportant également une sous-zone de phase (6b à 9b) en plus de la sous-zone principale (6a à 9a), les sous-zones de phase (6b à 9b) ayant des vergences de sous-zone de phase et des déphasages entre des ondes partielles de différentes zones annulaires (6 à 9) étant générés par les sous-zones de phase (6b à 9b), les sous-zones principales (6a à 9a) étant des structures annulaires qui s'étendent au moins partiellement autour de l'axe optique principal (A) et les sous-zones de phase (6b à 9b) étant des structures annulaires qui s'étendent au moins partiellement autour de l'axe optique principal (A), et une sous-zone principale (6a à 9a) étant directement adjacente, dans la direction radiale par rapport à l'axe optique principal (A), à une sous-zone de phase (6b à 9b) d'une zone annulaire (6 à 9), **caractérisée en ce que** la lentille intraoculaire (1) comporte en plus des zones annulaires (6 à 9) un enroulement hélicoïdal (12) comme structure de surface, une vergence de la lentille intraoculaire (1) variant en valeur dans le sens de rotation autour de l'axe principal (A) en raison de l'enroulement hélicoïdal (12), et l'enroulement hélicoïdal (12) étant formé sur au moins une face (4, 5), une vergence de sous-zone principale, qui contribue à la vergence totale de la lentille intraoculaire (1) par le biais de la sous-zone principale (6a à 9a), variant au moins une fois dans la direction azimutale autour de l'axe principal (A) et/ou une vergence de sous-zone de phase, qui contribue à la vergence totale de la lentille intraoculaire (1) par le biais de la sous-zone de phase (6b à 9b), variant au moins une fois dans la direction azimutale autour de l'axe principal (A), et une amplitude de phase (Φ) étant située entre deux zones adjacentes (6 à 9) dans l'intervalle < λ, en particulier dans l'intervalle 0,3 λ ≤ Φ ($\alpha$) ≤ 0,6 λ, l'amplitude de phase (Φ) variant dans la direction azimutale autour de l'axe principal (A) au moins dans une zone (6 à 9), l'amplitude de phase (Φ) étant égale à zéro sur une première partie d'intervalle d'azimut et étant différente de zéro sur une deuxième partie d'intervalle d'azimut et un rapport entre une vergence lointaine et une vergence proche de la lentille oculaire (1) pouvant être modifié en fonction du rapport entre la première et la deuxième partie d'intervalle d'azimut.

2. Lentille intraoculaire (1) selon la revendication 1, **caractérisée en ce que** les zones annulaires (6 à 9) et l'enroulement hélicoïdal (12) sont formées et superposées sur une face commune (4, 5).

3. Lentille intraoculaire (1) selon la revendication 1, **caractérisée en ce que** les zones annulaires (6 à 9) sont formées sur une face (4, 5) et l'enroulement hélicoïdal (12) est formé sur l'autre face (4, 5).

4. Lentille intraoculaire (1) selon l'une des revendications précédentes, **caractérisée en ce que** l'amplitude de phase (Φ) est constante au moins dans une zone (6 à 9) dans la direction azimutale autour de l'axe principal (A).

**5.** Lentille intraoculaire (1) selon l'une des revendications précédentes, **caractérisée en ce que** l'amplitude de phase (Φ) varie en continu.

**6.** Lentille intraoculaire (1) selon l'une des revendications 1 à 4 précédentes, **caractérisée en ce que** l'amplitude de phase (Φ) varie au moins une fois d'un niveau d'amplitude de phase discret.

**7.** Lentille intraoculaire (1) selon l'une des revendications précédentes, **caractérisée en ce que** l'enroulement hélicoïdal (12) est formé une fois autour de l'axe principal (A) et une brusque transition (13) est formée entre le début (14) de l'enroulement et la fin (15) de l'enroulement.

**8.** Lentille intraoculaire (1) selon l'une des revendications précédentes, **caractérisée en ce que**, en plus des zones annulaires (6 à 9) et de l'enroulement hélicoïdal (12), la lentille intraoculaire (1) présente une forme de surface (16) à réfraction torique, et la forme de surface (16) à réfraction torique est formée sur au moins une face (4, 5).

**9.** Lentille intraoculaire (1) selon les revendications 7 et 8, **caractérisée en ce que** la transition (13) est décalée dans la direction azimutale par rapport à un méridien principal plat (17, 17a, 17b) de la forme de surface (16) à réfraction torique, en particulier la brusque transition (13) est formée en position azimutale autour de l'axe optique principal (A) en formant un angle azimutal compris entre 20° et 70°, en particulier entre 40° et 50°, ou entre 200° et 250°, en particulier entre 220° et 230°, par rapport à un méridien principal plat (17, 17a, 17b) de la forme de surface (16) à réfraction torique, en particulier le décalage est compris entre $(1/4\ \pi)$ +/- $(1/8\ \pi)$ ou $(5/4\ \pi)$ +/- $(1/8\ \pi)$.

**10.** Lentille intraoculaire (1) selon l'une des revendications précédentes, **caractérisée en ce qu'**elle comporte $n \geq 2$ foyers et l'enroulement hélicoïdal (12) est conçu de sorte qu'un maximum de n-1 foyers soit augmenté en profondeur de champ par l'enroulement hélicoïdal (12).

**11.** Lentille intraoculaire (1) selon l'une des revendications précédentes, **caractérisée en ce qu'**une vergence totale de la lentille intraoculaire (1) varie dans la direction azimutale lors d'une révolution autour de l'axe principal (A) en augmentant sans minimums intermédiaires.

Fig.1a

Fig.1b

```
P0     = 20;
add    = 3.75;
n1     = 1.336;
n2     = 1.46;
ct     = 0.001;
λ      = 0.546;
Δλ     = 0.46;
k      = -2.75;
frac   = 0.85;
rm     = 3;
smax   = nring[Padd];
cyl    = 0.0;
sph    = 20.0;
edof   = 1.0;
HOA1   = 0.0;
HOA2   = 0.0;
```

## Fig.2a

F_Ges

## Fig.2b

F_EDoF (φ)

## Fig.2c

Fig.2d

Fig.2e

Fig.2f

Fig.2h

Fig.2g

```
P0    = 20;
add   = 2.0;
n1    = 1.336;
n2    = 1.46;
ct    = 0.001;
λ     = 0.546;
Δλ    = 0.46;
k     = -2.75;
frac  = 0.85;
rm    = 3;
smax  = nring[Padd];

cyl   = 0.0;
sph   = 20.0;
edof  = 1.25;
HOA1  = 0.0;
HOA2  = 0.0;
```

Fig.3a

Fig.3b

F_EDoF (φ)

## Fig.3c

## Fig.3d

## Fig.3e

## Fig.3f

Fig.3g

```
P0     = 20;
add    = 3.75;
n1     = 1.336;
n2     = 1.46;
ct     = 0.001;
λ      = 0.546;
Δλ     = 0.46;
k      = -2.75;
frac   = 0.85;
rm     = 3;
smax   = nring[Padd];
cyl    = 4.0;
sph    = 20.0;
edof   = 1.0;
HOA1   = 0.0;
HOA2   = 0.0;
```

# Fig.4a

# Fig.4b

# Fig.4c

Fig.4d

Fig.4e

```
P0    = 20;
add   = 2.0;
n1    = 1.336;
n2    = 1.46;
ct    = 0.001;
λ     = 0.546;
Δλ    = 0.46;
k     = -2.75;
frac  = 0.85;
rm    = 3;
smax  = nring[Padd];
cyl   = 4.0;
sph   = 20.0;
edof  = 0.0;
HOA1  = 0.0;
HOA2  = 0.0;
```

# Fig.5a

F_Ges

# Fig.5b

F_Cyl (φ)

# Fig.5c

Fig.5d

Fig.5e

Fig.5f

Fig.5g

```
P0      = 5;
add     = 3.75;
n1      = 1.336;
n2      = 1.46;
ct      = 0.001;
λ       = 0.546;

k       = -2.75;
frac    = 0.85;
rm      = 3;
smax    = nring[Padd];
cyl     = 0.0;
sph     = 5.0;
edof    = 0.0;
HOA1    = 0.0;
HOA2    = 0.0;
phasemin = 0.0;
phasemax = 0.6;
rmmin = 3.0;
rmmax = 3.0;
```

## Fig.6a

## Fig.6b

## Fig.6c

## Fig.6d

Fig.6e

B$_F$

Fig.6f

Fig.6g

```
PO      = 10;
add     = 3.75;
n1      = 1.336;
n2      = 1.46;
ct      = 0.001;
λ       = 0.546;

k       = -2.75;
frac    = 0.85;
rm      = 3;
smax    = nring[Padd];
cyl     = 0.0;
sph     = 10.0;
edof    = 0.0;
HOA1    = 0.0;
HOA2    = 0.0;
phasemin = 0.45;
phasemax = 0.46;
rmmin = 3.0;
rmmax = 5.0;
```

# Fig.7a

F_Ges

Fig.7b

F_Cyl (φ)

Fig.7c

Fig.7d

Fig.7e

Fig.7f

Fig.7g

$F_S$

Fig.7h

Fig.7i

```
P0    = 5;
add   = 3.75;
n1    = 1.336;
n2    = 1.46;
ct    = 0.001;
λ     = 0.546;
Δλ    = 0.46;
k     = -2.75;
frac  = 0.85;
rm    = 3;
smax  = nring[Padd];
cyl   = 0.0;
sph   = 5.0;
edof  = 0.0;
HOA1  = 0.0;
HOA2  = 0.0;
phasemin = 0.0;
phasemax = 1.0;
rmmin = 3.0;
rmmax = 3.0;
```

## Fig.8a

Fig.8b

Fig.8c

r_S [mm]

Azimut [rad]

## Fig.8d

6

S [mm]

r [mm]

6a 6b

## Fig.8e

Fig.8g

Fig.8f

```
PO      = 20;
add     = 3.75;
n1      = 1.336;
n2      = 1.46;
ct      = 0.001;
λ       = 0.546;
Δλ      = 0.46;
k       = -2.75;
frac    = 0.85;
rm      = 3;
smax    = nring[Padd];
cyl     = 4.0;
sph     = 20.0;
edof    = 0.0;
HOA1    = 0.0;
HOA2    = 0.0;
phasemin = 0.0;
phasemax = 1.0;
rmmin = 3.0;
rmmax = 3.0;
```

## Fig.9a

F_Ges

## Fig.9b

F_Cyl (φ)

## Fig.9c

φ [λ]

## Fig.9d

Fig.9e

Fig.9f

Fig.9g

Fig.9h

```
P0      = 20;
add     = 3.75;
n1      = 1.336;
n2      = 1.46;
ct      = 0.001;
λ       = 0.546;
Δλ      = 0.46;
k       = -2.75;
frac    = 0.85;
rm      = 3;
smax    = nring[Padd];
cyl     = 2.0;
sph     = 20.0;
edof    = 0.0;
HOA1    = 0.0;
HOA2    = 0.0;
phasemin = 0.45;
phasemax = 0.46;
rmmin   = 3.0;
rmmax   = 4.5;
```

# Fig.10a

Fig.10b

# Fig.10c

# Fig.10d

Fig.10e

Fig.10f

Fig.10g

```
P0      = 10;
add     = 3.75;
n1      = 1.336;
n2      = 1.46;
ct      = 0.001;
λ       = 0.546;


k       = -2.75;
frac    = 0.85;
rm      = 3;
smax    = nring[Padd];
cyl     = 2.0;
sph     = 10.0;
edof    = 0.0;
HOA1    = 0.0;
HOA2    = 0.0;
phasemin = 0.45;
phasemax = 0.46;
rmmin = 3.0;
rmmax = 3.25;
```

# Fig.11a

# Fig.11b

# Fig.11c

Fig.11d

Fig.11e

Fig.11f

Fig.11g

Fig.11h

```
P0      = 10;
add     = 3.75;
n1      = 1.336;
n2      = 1.46;
ct      = 0.001;
λ       = 0.546;

k       = -2.75;
frac    = 0.85;
rm      = 3;
smax    = nring[Padd];
cyl     = 2.0;
sph     = 10.0;
edof    = 0.0;
HOA1    = 0.0;
HOA2    = 0.0;
phasemin = 0.45;
phasemax = 0.46;
rmmin   = 3.0;
rmmax   = 3.5;
```

# Fig.12a

# Fig.12b

# Fig.12c

# Fig.12d

Fig.12e

Fig.12f

Fig.12g

```
P0      = 10;
add     = 3.75;
nl      = 1.336;
n2      = 1.46;
ct      = 0.001;
λ       = 0.546;

k       = -2.75;
frac    = 0.85;
rm      = 3;
smax    = nring[Padd];
cyl     = 2.0;
sph     = 10.0;
edof    = 0.0;
HOA1    = 0.0;
HOA2    = 0.0;
phasemin = 0.45;
phasemax = 0.46;
rmmin   = 3.0;
rmmax   = 3.75;
```

## Fig.13a

## Fig.13b

## Fig.13c

## Fig.13d

Fig.13e

Fig.13f

Fig.13g

```
P0       = 10;
add      = 3.75;
n1       = 1.336;
n2       = 1.46;
ct       = 0.001;
λ        = 0.546;

k        = -2.75;
frac     = 0.85;
rm       = 3;
smax     = nring[Padd];
cyl      = 2.0;
sph      = 10.0;
edof     = 0.0;
HOA1     = 0.0;
HOA2     = 0.0;
phasemin = 0.45;
phasemax = 0.46;
rmmin    = 3.0;
rmmax    = 3.75;
```

# Fig.14a

# Fig.14b

# Fig.14c

# Fig.14d

r<sub>S</sub> [mm]

## Fig.14e

S [mm]

## Fig.14f

F<sub>S</sub>

[mm]

## Fig.14g

Fig.14h

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 60016219 T2
- DE 102011114752 A1
- DE 102011101899 A1
- DE 102010018436 A1
- DE 102005028933 A1